# EUROPEAN PATENT APPLICATION

(11) **EP 4 649 938 A1**
(43) Date of publication of application: **19.11.2025**
(21) Application number: 24741598.7
(22) Date of filing: 12.01.2024
(51) Int. Cl.: A61K 6/80, A61K 6/15, A61K 6/802, A61K 6/818, A61K 6/836

(54) **DENTAL COMPOSITION**

(30) Priority: 13.01.2023 JP 2023004169
(71) Applicant: Kuraray Noritake Dental Inc., Kurashiki-shi, Okayama 710-0801 (JP)
(72) Inventor: MATSUURA Ryo, Tainai-shi, Niigata 959-2653 (JP)
(74) Representative: D Young & Co LLP
(86) International application number: PCT/JP2024/000673
(87) International publication number: WO 2024/150826

(57) **Abstract**

The present invention provides a dental composition that possesses excellent adhesive properties to tooth structure, in addition to high mechanical strength and excellent ease of polishing in its cured product, along with favorable paste properties. The present invention relates to a dental composition comprising a monomer (a) having an acidic group, a monomer (b) having no acidic group, a polymerization initiator (c), and a filler (d), wherein the filler (d) comprises an aggregate filler (d-1) in which primary particles are aggregated, the primary particles are metal oxides, and the aggregate filler (d-1) has a specific surface area of 5 m²/g or more and 60 m²/g or less as measured by a BET method.

## Description

### TECHNICAL FIELD

The present invention relates to dental compositions. More specifically, the invention relates to a dental composition that possesses not only high mechanical strength, excellent ease of polishing, and favorable paste properties but also high adhesive strength to tooth structure.

### BACKGROUND ART

Restorative filling materials such as filling composite resins and filling compomers, and crown restoration materials such as metal alloys, porcelain, and resin materials are typically used for the restoration of damaged tooth structures (enamel, dentin, and cementum) caused by caries and other dental defects. However, restorative filling materials and crown restoration materials (in this specification, these are also referred to collectively as "dental restoration materials") themselves do not generally possess adhesive properties to the tooth structure. Consequently, various adhesive systems with bonding agents have been used for the bonding of the tooth structure and dental restoration materials. An example of adhesive systems that are in common use is the acid etching (total etching) adhesive system, whereby the surfaces of tooth structure are etched with an acid etchant such as a phosphoric acid aqueous solution, and a bonding material, or an adhesive, is applied to adhere a dental restoration material to the tooth structure.

The self-etching adhesive system, on the other hand, is an example of adhesive systems that do not use acid etchants. In the past, the mainstream in such adhesive systems has been a two-step process in which a self-etching primer containing an acidic monomer, a hydrophilic monomer, and water is first applied to surfaces of tooth structure, and a bonding material containing a crosslinkable monomer and a polymerization initiator is applied without rinsing the surfaces with water. Another type of self-etching adhesive system that has come to be commonly used in recent years is the single-step adhesive system, which uses a one-part dental adhesive (one-part bonding material) that serves as both a self-etching primer and a bonding material. The typical monomer components of the one-part bonding material are acidic monomers, hydrophilic monomers, and crosslinkable monomers, and the one-part bonding material typically uses water and hydrophilic volatile organic solvents.

Recent years have seen the development of self-adhesive dental composite resins possessing adhesive properties in the dental composite resins themselves, and some of the compositions that have already been put into practical use eliminate the use of bonding materials to reduce the number of steps in restorative treatment procedures (Patent Literatures 1 to 3). Generally, bonding materials differ from dental composite resins (such as self-adhesive dental composite resins) in that the bonding material contains a solvent (such as water or organic solvents), and differs in filler content from dental composite resins.

Generally, self-adhesive dental composite resins present challenges concerning adhesive strength to tooth structure, compared to bonding materials. To address these challenges, for example, self-adhesive dental composite resins are disclosed that comprise a water-soluble photopolymerization initiator with a solubility in water of 10 g/L or more at 25°C, or an asymmetrical acrylamide·methacrylic acid ester compound, in order to increase the characteristic adhesive strength of bonding materials (Patent Literatures 4 and 5).

### CITATION LIST

### Patent Literature

| | |
|---|---|
| Patent Literature 1: | JP 2004-529946 T |
| Patent Literature 2: | JP 2017-105716 A |
| Patent Literature 3: | JP 2018-065831 A |
| Patent Literature 4: | WO2019/044815 |
| Patent Literature 5: | WO2015/190101 |

### SUMMARY OF INVENTION

### Technical Problem

There are inventions concerning the adhesive strength of self-adhesive dental composite resins to tooth structure, as disclosed in Patent Literatures 1 to 5. However, there is no disclosure concerning the ability to easily achieve the desired smoothness with short polishing time (hereinafter, also referred to simply as "ease of polishing")-an important characteristic for dental composite resins.

As a rule, the cured product tends to improve its ease of polishing and smoothness retention when, for example, a filler with an average particle diameter of 1 µm or less is used. However, the filler, when kneaded into the monomer, causes a significant viscosity increase in the paste, making it challenging to increase the filler content. This leads to problems such as a decrease in the mechanical strength of the cured product, or impaired handling properties such as the paste becoming sticky before polymerization.

When fillers with an average particle diameter greater than 1 µm are used, it is easier to increase the filler ratio in the polymerizable monomer, yielding a cured product with sufficient mechanical strength, along with high paste handling properties. A problem, however, is that achieving sufficient gloss is difficult even after the final polish.

For these reasons, it has been challenging to improve the mechanical strength and ease of polishing of the cured product while maintaining balance.

Additionally, studies by the present inventor revealed that a self-adhesive dental composite resin prepared using a filler with a small average particle diameter led to a serious viscosity increase in the paste, preventing an increase in filler content. Although the ease of polishing was excellent, the mechanical strength decreased, and the adhesive strength to tooth structure was weak.

As described above, difficulties remain in realizing a dental composition that possesses excellent adhesive properties to tooth structure, in addition to high mechanical strength and excellent ease of polishing in its cured product, along with favorable paste properties.

It is accordingly an object of the present invention to provide a dental composition that possesses excellent adhesive properties to tooth structure, in addition to high mechanical strength and excellent ease of polishing in its cured product, along with favorable paste properties.

### Solution to Problem

The present inventor conducted intensive studies, and found that the foregoing issues can be solved with a dental composition that comprises a specific aggregate filler. This led to the completion of the present invention after further examinations.

Specifically, the present invention includes the following.
[1] A dental composition comprising a monomer (a) having an acidic group, a monomer (b) having no acidic group, a polymerization initiator (c), and a filler (d),
   wherein the filler (d) comprises an aggregate filler (d-1) in which primary particles are aggregated,
   the primary particles are metal oxides, and
   the aggregate filler (d-1) has a specific surface area of 5 m²/g or more and 60 m²/g or less as measured by a BET method.
[2] The dental composition according to [1], wherein the primary particles of the aggregate filler (d-1) have an average particle diameter of 30 to 500 nm.
[3] The dental composition according to [1] or [2], which has a viscosity of 1 to less than 200 Pa·s as measured with a rotational viscoelasticity meter at 25°C with a shear rate of 10 s⁻¹.
[4] The dental composition according to any one of [1] to [3], wherein the filler (d) further comprises a filler (d-2) other than the aggregate filler (d-1).
[5] The dental composition according to any one of [1] to [4], wherein the metal oxides forming the primary particles of the aggregate filler (d-1) are metal oxides comprise Si and Zr elements.
[6] The dental composition according to any one of [1] to [5], wherein the polymerization initiator (c) comprises a water-soluble photopolymerization initiator (c-1a).
[7] The dental composition according to any one of [1] to [6], wherein the content of the aggregate filler (d-1) is 20 to 85 mass% in total 100 mass% of the dental composition.
[8] The dental composition according to any one of [1] to [7], wherein the content of the monomer (a) having an acidic group is 0.1 to 25 mass% in total 100 mass% of the dental composition.
[9] The dental composition according to any one of [1] to [8], wherein the content of the filler (d) is 50 to 90 mass% in total 100 mass% of the dental composition.
[10] The dental composition according to any one of [1] to [9], wherein the aggregate filler (d-1) has an average particle diameter of 1 to 20 µm in terms of secondary particles.
[11] The dental composition according to any one of [1] to [10], wherein the aggregate filler (d-1) has a specific surface area of 5 m²/g or more and less than 50 m²/g as measured by a BET method.
[12] A self-adhesive dental composite resin comprising a dental composition of any one of [1] to [11].
[13] A dental cement comprising a dental composition of any one of [1] to [11].
[14] A method for producing a dental composition of any one of [1] to [10], comprising mixing at least three of the monomer (a) having an acidic group, the monomer (b) having no acidic group, the polymerization initiator (c), and the aggregate filler (d-1).
[15] The method for producing a dental composition according to [14], which further comprises mixing a filler (d-2) other than the aggregate filler (d-1), and a polymerization accelerator (e).

### Advantageous Effects of Invention

According to the present invention, a dental composition can be provided that possesses excellent adhesive properties to tooth structure, in addition to high mechanical strength and excellent ease of polishing in its cured product, along with favorable paste properties.

Specifically, the present invention can provide a dental composition that, because of excellent ease of polishing, makes it possible to easily achieve desired smoothness with less than 20 seconds of polishing.

The present invention can also achieve high mechanical strength and excellent ease of polishing in the cured product even when the filler content is high, in addition to providing favorable paste properties and excellent adhesive properties to tooth structure.

### DESCRIPTION OF EMBODIMENTS

A dental composition of the present invention comprises a monomer (a) having an acidic group, a monomer (b) having no acidic group, a polymerization initiator (c), and a filler (d).

The filler (d) comprises an aggregate filler (d-1) in which metal oxide primary particles are aggregated.

The aggregate filler (d-1) has a specific surface area of 5 m²/g or more and 60 m²/g or less as measured by a BET method.

Preferably, the secondary particles have an average particle diameter of 1 to 20 µm as measured by a laser diffraction method.

The term "(meth)acryl" as used in the present specification collectively refers to methacryl and acryl. The same applies to similar expressions (such as (meth)acrylic acid, and (meth)acrylonitrile).

In this specification, the upper limits and lower limits of numeric ranges (for example, ranges of contents of components, ranges of values calculated from components, and ranges of physical properties) can be appropriately combined.

In this specification, various modifications can be made to the embodiments, such as by appropriately combining part or all of the embodiments, except in specific cases where it is not applicable.

The inclusion of an aggregate filler having a certain specific surface area with aggregated metal oxide primary particles has enabled the balancing of excellent ease of polishing (typically more pronounced with smaller particle sizes) and mechanical strength (generally enhanced with larger particle sizes), in addition to achieving favorable paste properties, and, rather unexpectedly, excellent adhesive properties to tooth structure.

The following provides a possible explanation for why a dental composition of the present invention can exhibit ease of polishing and other desirable properties along with excellent adhesive properties to tooth structure.

For dental materials to exhibit adhesive properties to tooth structure, the monomer components must make contact with the tooth structure.

However, in compositions containing a large amount of filler (e.g., a filler content of 50 mass% or more) in the entire composition, the monomer components are less likely to contact the tooth structure compared to low-viscosity dental adhesives containing hardly any filler, which makes it difficult to achieve sufficient adhesive properties to tooth structure.

Furthermore, when fillers with small average particle diameters are used in large amounts in the composition to achieve the desired surface properties (for example, such as smoothness) in the cured product with short polishing time, a further decline in adhesive properties to tooth structure will probably occur due to reduced flowability of the composition upon mixing the fillers with the monomer component, and the resulting failure of the monomer component to sufficiently make contact with the tooth structure.

From the perspective of enhancing adhesive properties to tooth structure by increasing the amount of monomer component that can contact the tooth structure, it is therefore necessary to increase the particle size of the filler to improve its flowability.

However, it was found that, when the particle size of the filler is large, it is not easy to achieve the desired surface properties (for example, such as smoothness) with short polishing time when the composition contains a monomer that exhibits adhesive properties to tooth structure, though this produces excellent adhesive properties to tooth structure.

In contrast, a dental composition of the present invention comprises an aggregate filler having a certain BET specific surface area, with aggregated metal oxide primary particles in the filler.

In this case, the presence of the monomer component in the spaces between the primary particles can be ignored, and the flowability of the monomer that exhibits adhesive properties to tooth structure is affected by the BET specific surface area of the aggregate filler formed by the aggregation of primary particles.

Because smaller BET specific surface areas lead to greater flowability of the monomer component, the monomer that exhibits adhesive properties to tooth structure becomes more likely to make contact with the tooth structure. This has probably led to the development of excellent adhesive properties to tooth structure.

It is also presumed that using the aggregate filler has enabled the balancing with ease of polishing.

The following describes the components used in a dental composition of the present invention.

### <Monomer (a) Having Acidic Group>

In view of imparting excellent adhesive properties to tooth structure, the dental composition comprises a monomer (a) having an acidic group.

By incorporating a monomer (a) having an acidic group, it is possible to achieve high adhesive strength to tooth structure. Preferred for use in the dental composition are radical polymerizable monomers.

Specific examples of radical polymerizable monomers in monomer (a) having an acidic group include (meth)acrylate monomers, (meth)acrylamide monomers, esters, vinyl esters, and vinyl ethers of acids such as α-cyanoacrylic acid, (meth)acrylic acid, α-halogenated acrylic acid, crotonic acid, cinnamic acid, sorbic acid, maleic acid, and itaconic acid, and mono-N-vinyl derivatives and styrene derivatives. In view of curability, preferred are (meth)acrylate monomers and (meth)acrylamide monomers.

Examples of the monomer (a) having an acidic group used in the present invention include monomers having at least one acidic group such as a phosphoric acid group, a pyrophosphoric acid group, a thiophosphoric acid group, a phosphonic acid group, a carboxylic acid group, and a sulfonic acid group.

The monomer (a) having an acidic group may be used alone, or two or more thereof may be used in appropriate combinations. Specific examples of the monomer (a) having an acidic group are as follows.

Examples of monomers having a phosphoric acid group include 2-(meth)acryloyloxyethyl dihydrogen phosphate, 3-(meth)acryloyloxypropyl dihydrogen phosphate, 4-(meth)acryloyloxybutyl dihydrogen phosphate, 5-(meth)acryloyloxypentyl dihydrogen phosphate, 6-(meth)acryloyloxyhexyl dihydrogen phosphate, 7-(meth)acryloyloxyheptyl dihydrogen phosphate, 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, 20-(meth)acryloyloxyicosyl dihydrogen phosphate, bis[2-(meth)acryloyloxyethyl]hydrogen phosphate, bis[4-(meth)acryloyloxybutyl]hydrogen phosphate, bis[6-(meth)acryloyloxyhexyl]hydrogen phosphate, bis[8-(meth)acryloyloxyoctyl]hydrogen phosphate, bis[9-(meth)acryloyloxynonyl]hydrogen phosphate, bis[10-(meth)acryloyloxydecyl]hydrogen phosphate, 1,3-di(meth)acryloyloxypropyl-2-dihydrogen phosphate, 2-(meth)acryloyloxyethylphenyl hydrogen phosphate, 2-(meth)acryloyloxyethyl-(2-bromoethyl)hydrogen phosphate, 2-methacryloyloxyethyl-(4-methoxyphenyl)hydrogen phosphate, 2-methacryloyloxypropyl-(4-methoxyphenyl)hydrogen phosphate, and acid chlorides, alkali metal salts, and amine salts of these. Preferred are monomers having divalent phosphoric acid groups with C6 to C12 alkylene groups.

A certain preferred embodiment is, for example, a self-adhesive dental composite resin in which the monomer (a) having an acidic group comprises a monomer having a divalent phosphoric acid group with a C6 to C12 alkylene group.

Examples of monomers having a pyrophosphoric acid group include bis[2-(meth)acryloyloxyethyl]pyrophosphate, bis[4-(meth)acryloyloxybutyl]pyrophosphate, bis[6-(meth)acryloyloxyhexyl]pyrophosphate, bis[8-(meth)acryloyloxyoctyl]pyrophosphate, bis[10-(meth)acryloyloxydecyl]pyrophosphate, and acid chlorides, alkali metal salts, and amine salts of these.

Examples of monomers having a thiophosphoric acid group include 2-(meth)acryloyloxyethyl dihydrogen thiophosphate, 3-(meth)acryloyloxypropyl dihydrogen thiophosphate, 4-(meth)acryloyloxybutyl dihydrogen thiophosphate, 5-(meth)acryloyloxypentyl dihydrogen thiophosphate, 6-(meth)acryloyloxyhexyl dihydrogen thiophosphate, 7-(meth)acryloyloxyheptyl dihydrogen thiophosphate, 8-(meth)acryloyloxyoctyl dihydrogen thiophosphate, 9-(meth)acryloyloxynonyl dihydrogen thiophosphate, 10-(meth)acryloyloxydecyl dihydrogen thiophosphate, 11-(meth)acryloyloxyundecyl dihydrogen thiophosphate, 12-(meth)acryloyloxydodecyl dihydrogen thiophosphate, 16-(meth)acryloyloxyhexadecyl dihydrogen thiophosphate, 20-(meth)acryloyloxyicosyl dihydrogen thiophosphate, and acid chlorides, alkali metal salts, and ammonium salts of these.

Examples of monomers having a phosphonic acid group include 2-(meth)acryloyloxyethylphenyl phosphonate, 5-(meth)acryloyloxypentyl-3-phosphonopropionate, 6-(meth)acryloyloxyhexyl-3-phosphonopropionate, 10-(meth)acryloyloxydecyl-3-phosphonopropionate, 6-(meth)acryloyloxyhexyl phosphonoacetate, 10-(meth)acryloyloxydecyl-3-phosphonoacetate, and acid chlorides, alkali metal salts, and ammonium salts of these.

Examples of monomers having a carboxylic acid group include (meth)acrylic acid esters having one carboxyl group or acid anhydride group thereof per molecule, and (meth)acrylic acid esters having a plurality of carboxyl groups or acid anhydride groups thereof per molecule.

Examples of monomers having one carboxyl group or acid anhydride group thereof per molecule include (meth)acrylic acid, N-(meth)acryloylglycine, N-(meth)acryloylaspartic acid, 2-(meth)acryloyloxyethyl hydrogen succinate, 2-(meth)acryloyloxyethyl hydrogen phthalate, 2-(meth)acryloyloxyethyl hydrogen malate, O-(meth)acryloyltyrosine, N-(meth)acryloyltyrosine, N-(meth)acryloylphenylalanine, N-(meth)acryloyl-p-aminobenzoic acid, N-(meth)acryloyl-o-aminobenzoic acid, 2-(meth)acryloyloxybenzoic acid, 3-(meth)acryloyloxybenzoic acid, 4-(meth)acryloyloxybenzoic acid, N-(meth)acryloyl-5-aminosalicylic acid, and N-(meth)acryloyl-4-aminosalicylic acid, and compounds derived by converting the carboxyl group of the aforementioned compounds into an acid anhydride group.

Examples of monomers having a plurality of carboxyl groups or acid anhydride groups thereof per molecule include 6-(meth)acryloyloxyhexane-1, 1-dicarboxylic acid, 9-(meth)acryloyloxynonane-1,1-dicarboxylic acid, 10-(meth)acryloyloxydecane-1,1-dicarboxylic acid, 11-(meth)acryloyloxyundecane-1,1-dicarboxylic acid, 12-(meth)acryloyloxydodecane-1,1-dicarboxylic acid, 13-(meth)acryloyloxytridecane-1,1-dicarboxylic acid, 4-(meth)acryloyloxyethyltrimellitate, 4-(meth)acryloyloxyethyltrimellitate anhydride, 4-(meth)acryloyloxybutyltrimellitate, 4-(meth)acryloyloxyhexyltrimellitate, 4-(meth)acryloyloxydecyltrimellitate, 2-(meth)acryloyloxyethyl-3'-(meth)acryloyloxy-2'-(3,4-dicarboxybenzoyloxy)propylsuccinate, 6-(meth)acryloyloxyethylnaphthalene-1,2,6-tricarboxylic acid anhydride, 6-(meth)acryloyloxyethylnaphthalene-2,3,6-tricarboxylic acid anhydride, 4-(meth)acryloyloxyethylcarbonylpropionoyl-1,8-naphthalic acid anhydride, and 4-(meth)acryloyloxyethylnaphthalene-1,8-tricarboxylic acid anhydride.

Examples of monomers having a sulfonic acid group include 2-sulfoethyl (meth)acrylate.

In view of providing good adhesive strength for applications as self-adhesive dental composite resins, it is preferable that the monomer (a) having an acidic group include monomers having a phosphoric acid group, or monomers having a carboxylic acid group, more preferably 2-(meth)acryloyloxyethyl dihydrogen phosphate, 3-(meth)acryloyloxypropyl dihydrogen phosphate, 4-(meth)acryloyloxybutyl dihydrogen phosphate, 5-(meth)acryloyloxypentyl dihydrogen phosphate, 6-(meth)acryloyloxyhexyl dihydrogen phosphate, 7-(meth)acryloyloxyheptyl dihydrogen phosphate, 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, 20-(meth)acryloyloxyicosyl dihydrogen phosphate, 4-(meth)acryloyloxyethyltrimellitate anhydride, 4-(meth)acryloyloxyethyltrimellitate, 11-(meth)acryloyloxyundecane-1,1-dicarboxylic acid, and a mixture of 2-methacryloyloxyethyl dihydrogen phosphate and bis(2-methacryloyloxyethyl)hydrogen phosphate, even more preferably 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, and 20-(meth)acryloyloxyicosyl dihydrogen phosphate. In view of a balance with curability, 10-(meth)acryloyloxydecyl dihydrogen phosphate is particularly preferred.

In view of adhesive properties to tooth structure, the content of the monomer (a) having an acidic group in the dental composition is preferably 1 to 40 parts by mass, more preferably 2 to 35 parts by mass, even more preferably 3 to 30 parts by mass, particularly preferably 5 to 25 parts by mass in total 100 parts by mass of monomers in the dental composition.

In certain embodiments, in view of adhesive properties to tooth structure, the content of the monomer (a) having an acidic group is preferably 0.1 mass% or more, more preferably 0.2 mass% or more, even more preferably 0.5 mass% or more, particularly preferably 1 mass% or more in total 100 mass% of the dental composition.

In view of adhesive properties to tooth structure, the content of the monomer (a) having an acidic group is preferably 25 mass% or less, more preferably 20 mass% or less, even more preferably 15 mass% or less, particularly preferably 10 mass% or less in total 100 mass% of the dental composition.

### <Monomer (b) Having no Acidic Group>

Examples of the monomer (b) having no acidic group in the present invention include a hydrophobic monomer (b-1) having no acidic group and having a solubility of less than 10 mass% in 25°C water (hereinafter, also referred to simply as "hydrophobic monomer (b-1)"), and a hydrophilic monomer (b-2) having no acidic group and having a solubility of 10 mass% or more in 25°C water (hereinafter, also referred to simply as "hydrophilic monomer (b-2)").

The monomer (b) having no acidic group may be used alone, or two or more thereof may be used in combination.

### · Hydrophobic Monomer (b-1) Having no Acidic Group

The hydrophobic monomer (b-1) having no acidic group improves properties such as handling of the dental composition, and the mechanical strength (flexural strength) of the cured product, and reduces water absorption and dissolution.

The hydrophobic monomer (b-1) is preferably a radical polymerizable monomer having no acidic group but having a polymerizable group. In view of ease of radical polymerization, preferred as the polymerizable group are (meth)acryloyloxy groups and/or (meth)acrylamide groups.

The hydrophobic monomer (b-1) refers to a monomer that has no acidic group, and that has a solubility of less than 10 mass% in 25°C water.

The hydrophobic monomer (b-1) can be classified into monofunctional hydrophobic monomers (for example, (meth)acrylate monomers, (meth)acrylamide monomers), aromatic bifunctional hydrophobic monomers, aliphatic bifunctional hydrophobic monomers, and tri- and higher-functional hydrophobic monomers.

The hydrophobic monomer (b-1) may be used alone, or two or more thereof may be used in combination.

Examples of the monofunctional hydrophobic monomers include:
aliphatic monofunctional (meth)acrylate monomers such as n-stearyl methacrylate;
aliphatic monofunctional (meth)acrylate monomers containing an ether bond(s), such as butoxydiethylene glycol methacrylate, and methoxypolyethylene glycol methacrylate (with an average of nine moles of oxyethylene group added);
alicyclic monofunctional (meth)acrylate monomers such as cyclohexyl methacrylate, isobornyl methacrylate, and dicyclopentanyl methacrylate;
monofunctional (meth)acrylate monomers having an aromatic ring group(s), such as benzyl methacrylate (commonly known as BEMA), 2-phenoxyethyl methacrylate (commonly known as PEMA), phenoxybenzyl methacrylate (commonly known as POB-MA), 1-naphthylmethyl (meth)acrylate, and 2-naphthylmethyl (meth)acrylate; and
(meth)acrylate monomers containing a heterocyclic ring group(s) (for example, cyclic ether groups), such as tetrahydrofurfuryl methacrylate (commonly known as THF-MA).

The monofunctional (meth)acrylate monomers having an aromatic ring group(s) are preferably those containing one or two phenyl groups.

The (meth)acrylate monomers containing a heterocyclic ring group(s) are preferably those containing one or two heterocyclic ring groups (for example, cyclic ether groups).

Examples of the aromatic bifunctional hydrophobic monomers include 2,2-bis((meth)acryloyloxyphenyl)propane, 2,2-bis[4-(2-hydroxy-3-(meth)acryloyloxypropoxy)phenyl]propane, 2,2-bis(4-(meth)acryloyloxyethoxyphenyl)propane, 2,2-bis(4-(meth)acryloyloxypolyethoxyphenyl)propane, 2,2-bis(4-(meth)acryloyloxydiethoxyphenyl)propane, 2,2-bis(4-(meth)acryloyloxytriethoxyphenyl)propane, 2,2-bis(4-(meth)acryloyloxytetraethoxyphenyl)propane, 2,2-bis(4-(meth)acryloyloxypentaethoxyphenyl)propane, 2,2-bis(4-(meth)acryloyloxydipropoxyphenyl)propane, 2-(4-(meth)acryloyloxydiethoxyphenyl)-2-(4-(meth)acryloyloxyethoxyphenyl)propane, 2-(4-(meth)acryloyloxydiethoxyphenyl)-2-(4-(meth)acryloyloxytriethoxyphenyl)propane, 2-(4-(meth)acryloyloxydipropoxyphenyl)-2-(4-(meth)acryloyloxytriethoxyphenyl)propane, 2,2-bis(4-(meth)acryloyloxypropoxyphenyl)propane, and 2,2-bis(4-(meth)acryloyloxyisopropoxyphenyl)propane.

In view of mechanical strength and handling properties, preferred among these are 2,2-bis[4-(2-hydroxy-3-methacryloyloxypropoxy)phenyl]propane (commonly known as Bis-GMA), 2,2-bis(4-(meth)acryloyloxyethoxyphenyl)propane, 2,2-bis(4-methacryloyloxypolyethoxyphenyl)propane (with an average of 2.6 moles of ethoxy group added; commonly known as D-2.6E), 2,2-bis(4-(meth)acryloyloxydiethoxyphenyl)propane, 2,2-bis(4-(meth)acryloyloxytriethoxyphenyl)propane, 2,2-bis(4-(meth)acryloyloxytetraethoxyphenyl)propane, and 2,2-bis(4-(meth)acryloyloxypentaethoxyphenyl)propane.

Examples of the aliphatic bifunctional hydrophobic monomers include glycerol di(meth)acrylate, ethylene glycol di(meth)acrylate, diethylene glycol di(meth)acrylate, triethylene glycol di(meth)acrylate, propylene glycol di(meth)acrylate, butylene glycol di(meth)acrylate, neopentyl glycol di(meth)acrylate, 1,3-butanediol di(meth)acrylate, 1,5-pentanediol di(meth)acrylate, 1,6-hexanediol di(meth)acrylate, 1,10-decanediol di(meth)acrylate, 1,2-bis(3-methacryloyloxy-2-hydroxypropoxy)ethane, 2,2,4-trimethylhexamethylenebis(2-carbamoyloxyethyl)di(meth)acrylate, N-methacryloyloxyethylacrylamide (commonly known as MAEA), N-methacryloyloxypropylacrylamide, N-methacryloyloxybutylacrylamide, N-(1-ethyl-(2-methacryloyloxy)ethyl)acrylamide, and N-(2-(2-methacryloyloxyethoxy)ethyl)acrylamide.

In view of mechanical strength and handling properties, preferred are triethylene glycol diacrylate, triethylene glycol dimethacrylate (commonly known as 3G), neopentyl glycol di(meth)acrylate, 1,2-bis(3-methacryloyloxy-2-hydroxypropoxy)ethane, 2,2,4-trimethylhexamethylenebis(2-carbamoyloxyethyl)dimethacrylate (commonly known as UDMA), 1,10-decanediol dimethacrylate (commonly known as DD), and 2,2,4-trimethylhexamethylenebis(2-carbamoyloxyethyl)dimethacrylate. Preferred in view of polymerization shrinkage stress are 1,2-bis(3-methacryloyloxy-2-hydroxypropoxy)ethane, UDMA, and DD. MAEA, and N-methacryloyloxypropylacrylamide are preferred in view of adhesive properties to tooth structure, particularly dentin.

Examples of the tri- and higher-functional hydrophobic monomers include trimethylolpropane tri(meth)acrylate, trimethylolethane tri(meth)acrylate, trimethylolmethane tri(meth)acrylate, pentaerythritol tri(meth)acrylate, pentaerythritol tetra(meth)acrylate, dipentaerythritol penta(meth)acrylate, N,N-(2,2,4-trimethylhexamethylene)bis[2-(aminocarboxy)propane-1,3-diol]tetra(meth)acrylate, and 1,7-diacryloyloxy-2,2,6,6-tetra(meth)acryloyloxymethyl-4-oxaheptane. In view of mechanical strength, N,N-(2,2,4-trimethylhexamethylene)bis[2-(aminocarboxy)propane-1,3-diol]tetramethacrylate is preferred.

In view of the mechanical strength (flexural strength), water absorption, dissolution, and handling properties, preferred for use among these hydrophobic monomers (b-1) are aromatic bifunctional hydrophobic monomers, aliphatic bifunctional hydrophobic monomers, and monofunctional hydrophobic monomers.

Preferred as aromatic bifunctional monomers are Bis-GMA and D-2.6E.

Preferred as aliphatic bifunctional monomers are 3G, neopentyl glycol di(meth)acrylate, UDMA, DD, and MAEA.

Preferred as monofunctional hydrophobic monomers are 1-naphthylmethyl methacrylate (commonly known as 1-NMMA), THF-MA, BEMA, POB-MA, and PEMA.

In view of mechanical strength and adhesive properties to tooth structure, it is preferable for applications as dental compositions that the hydrophobic monomer (b-1) be a hydrophobic monomer with no hydroxyl group (a monofunctional hydrophobic monomer with no hydroxyl group, an aromatic bifunctional hydrophobic monomer with no hydroxyl group, or an aliphatic bifunctional hydrophobic monomer with no hydroxyl group), more preferably D-2.6E, DD, UDMA, MAEA, THF-MA, BEMA, POB-MA, or PEMA, even more preferably D-2.6E, DD, MAEA, or THF-MA.

The content of the hydrophobic monomer (b-1) in the dental composition is preferably 20 to 98 parts by mass, more preferably 40 to 95 parts by mass, even more preferably 60 to 92 parts by mass, particularly preferably 60 to 90 parts by mass in total 100 parts by mass of monomers in the dental composition.

When the content of hydrophobic monomer (b-1) is at or below these upper limits, a decrease of adhesive properties due to reduced wettability of the dental composition to tooth structure can more easily be reduced, whereas the cured product can more easily exhibit the desired mechanical strength and handling properties when the content of hydrophobic monomer (b-1) is at or above the foregoing lower limits.

In certain embodiments, in view of adhesive properties to tooth structure, the mechanical strength of the cured product, and handling properties, the content of hydrophobic monomer (b-1) is preferably 5 mass% or more, more preferably 8 mass% or more, even more preferably 10 mass% or more, particularly preferably 15 mass% or more in total 100 mass% of the dental composition.

In view of adhesive properties to tooth structure, the mechanical strength of the cured product, and handling properties, the content of hydrophobic monomer (b-1) is preferably 45 mass% or less, more preferably 40 mass% or less, even more preferably 35 mass% or less, particularly preferably 30 mass% or less in total 100 mass% of the dental composition.

### · Hydrophilic Monomer (b-2) Having no Acidic Group

The hydrophilic monomer (b-2) can enhance the adhesive strength to tooth structure by improving the wettability for applications as self-adhesive dental composite resin to tooth structure, and the penetration or applications as self-adhesive dental composite resin into tooth structure (enamel/dentin).

The hydrophilic monomer (b-2) is preferably a radical polymerizable monomer having no acidic group but having a polymerizable group. In view of ease of radical polymerization, the polymerizable group is preferably a (meth)acryloyloxy group and/or a (meth)acrylamide group.

The hydrophilic monomer (b-2) refers to a monomer that has no acidic group, and that has a solubility of 10 mass% or more in 25°C water. The hydrophilic monomer (b-2) is preferably one having a solubility of 30 mass% or more in 25°C water, more preferably one that can dissolve in water in any proportions at 25°C.

The hydrophilic monomer is preferably one having a hydrophilic group such as a hydroxyl group, an oxymethylene group, an oxyethylene group, an oxypropylene group, or an amide group.

Examples of the hydrophilic monomer (b-2) include hydrophilic monofunctional (meth)acrylate monomers such as 2-hydroxyethyl (meth)acrylate, 3-hydroxypropyl (meth)acrylate, 2-hydroxypropyl (meth)acrylate, 1,3-dihydroxypropyl (meth)acrylate, 2,3-dihydroxypropyl (meth)acrylate, 2-((meth)acryloyloxy)ethyltrimethylammonium chloride, and polyethylene glycol di(meth)acrylate (with an average of nine or more moles of oxyethylene group added); and hydrophilic monofunctional (meth)acrylamide monomers such as N-methylol(meth)acrylamide, N-hydroxyethyl(meth)acrylamide, N,N-bis(2-hydroxyethyl)(meth)acrylamide, N-methoxymethyl(meth)acrylamide, N-ethoxymethyl(meth)acrylamide, diacetone (meth)acrylamide, 4-(meth)acryloylmorpholine, N-trihydroxymethyl-N-methyl(meth)acrylamide, N,N-dimethylacrylamide, and N,N-diethylacrylamide.

In view of adhesive properties to tooth structure, preferred as hydrophilic monomer (b-2) are 2-hydroxyethyl (meth)acrylate, 2,3-dihydroxypropyl (meth)acrylate, and hydrophilic monofunctional (meth)acrylamide monomers, more preferably 2-hydroxyethyl (meth)acrylate, N,N-dimethylacrylamide, and N,N-diethylacrylamide.

The hydrophilic monomer (b-2) may be incorporated alone, or two or more thereof may be used in combination.

The content of the hydrophilic monomer (b-2) in the dental composition is preferably 0 to 50 parts by mass, more preferably 0 to 40 parts by mass, even more preferably 0 to 30 parts by mass, particularly preferably 0 to 25 parts by mass in total 100 parts by mass of the monomers. The content of hydrophilic monomer (b-2) may be 0 parts by mass in total 100 parts by mass of the monomers.

When the content of the hydrophilic monomer (b-2) in the dental composition is at or above these lower limits, it is easier to produce an effect that sufficiently improves the adhesive strength, whereas the cured product can more easily exhibit the desired mechanical strength when the content of hydrophilic monomer (b-2) is at or below the foregoing upper limits.

### [(Meth)Acrylic Compound (b-3) Having Weight-Average Molecular Weight of 1,000 to 80,000, and Weight-Average Molecular Weight of 1,250 or More and Less Than 20,000 per Polymerizable Group]

In a dental composition of the present invention, a (meth)acrylic compound (b-3) having a weight-average molecular weight of 1,000 to 80,000, and a weight-average molecular weight of 1,250 or more and less than 20,000 per polymerizable group (hereinafter, referred to as (meth)acrylic compound (b-3)) may be used to impart low polymerization shrinkage stress. In the context of monomer (b) having no acidic group, (meth)acrylic compounds with a weight-average molecular weight of 1,000 to 80,000, and a weight-average molecular weight of 1,250 or more and less than 20,000 per polymerizable group are classified as (meth)acrylic compounds (b-3) in this specification, irrespective of the solubility in 25°C water.

The (meth)acrylic compound (b-3) can be broadly classified into urethanized (meth)acrylic compound (b-3a) and (meth)acrylic compound (b-3b) having no urethane skeleton.

The urethanized (meth)acrylic compound (b-3a) is preferred in view of ease of introducing a (meth)acryl group, and the effect to reduce polymerization shrinkage stress. The urethanized (meth)acrylic compound (b-3a) can be easily synthesized by, for example, an addition reaction of a polyol containing a polymer skeleton (described later), a compound having an isocyanate group (-NCO), and a (meth)acrylic compound having a hydroxyl group (-OH).

Alternatively, the urethanized (meth)acrylic compound (b-3a) can be synthesized with ease by allowing lactone or alkylene oxide to undergo a ring-opening addition reaction with a (meth)acrylic compound having a hydroxyl group, and causing the resulting compound having a terminal hydroxyl group to undergo an addition reaction with a compound having an isocyanate group. The (meth)acrylic compound (b-3b) having no urethane skeleton can be obtained, for example, through a dehydrocondensation reaction of (meth)acrylic acid with a polymer of a monomer having a hydroxyl group.

### · Urethanized (Meth)Acrylic Compound (b-3a)

The urethanized (meth)acrylic compound (b-3a) is preferably a (meth)acrylate having a structure (a polymer skeleton) selected from the group consisting of a polyester, a polycarbonate, a polyurethane, a polyether, a poly-conjugated diene, and a hydrogenated poly-conjugated diene, in addition to a urethane bond. More preferably, the urethanized (meth)acrylic compound (b-3a) is a (meth)acrylate having at least one polyol moiety, per molecule, selected from the group consisting of a polyester, a polycarbonate, a polyurethane, a polyether, a poly-conjugated diene, and a hydrogenated poly-conjugated diene each having a structure derived from a C4 to C18 aliphatic diol unit having a branched structure.

In such structures, examples of the polyester include: a copolymer of a dicarboxylic acid (e.g., an aromatic dicarboxylic acid such as phthalic acid or isophthalic acid, or an unsaturated aliphatic dicarboxylic acid such as maleic acid) and an aliphatic diol having 2 to 18 carbon atoms; a copolymer of a dicarboxylic acid (e.g., a saturated aliphatic dicarboxylic acid such as adipic acid or sebacic acid) and an aliphatic diol having 2 to 18 carbon atoms; a polymer of β-propiolactone; a polymer of γ-butyrolactone; a polymer of δ-valerolactone; a polymer of ε-caprolactone; and a copolymer of these. Preferred are a copolymer of a dicarboxylic acid (an aromatic dicarboxylic acid such as phthalic acid or isophthalic acid, or an unsaturated aliphatic dicarboxylic acid such as maleic acid) and an aliphatic diol having 2 to 12 carbon atoms; and a copolymer of a dicarboxylic acid (a saturated aliphatic dicarboxylic acid such as adipic acid or sebacic acid) and an aliphatic diol having 2 to 12 carbon atoms.

Examples of the polycarbonate include polycarbonates derived from aliphatic diols having 2 to 18 carbon atoms, polycarbonates derived from bisphenol A, and polycarbonates derived from C2 to C18 aliphatic diols and bisphenol A. Preferred are polycarbonates derived from aliphatic diols having 2 to 12 carbon atoms, polycarbonates derived from bisphenol A, and polycarbonates derived from C2 to C12 aliphatic diols and bisphenol A.

Examples of the polyurethane include a polymer of a C2 to C18 aliphatic diol and a C1 to C18 diisocyanate. Preferred is a polymer of a C2 to C12 aliphatic diol and a C1 to C12 diisocyanate.

Examples of the polyether include polyethylene glycol, polypropylene glycol, polybutylene glycol, and poly(1-methylbutylene glycol).

Examples of the poly-conjugated diene and hydrogenated poly-conjugated diene include 1,4-polybutadiene, 1,2-polybutadiene, polyisoprene, poly(butadiene-isoprene), poly(butadiene-styrene), poly(isoprene-styrene), polyfarnesene, and hydrogenated products of these. In view of superior mechanical strength and water resistance, preferred among these are the polyester, polycarbonate, and poly-conjugated diene structures, more preferably the structures of polyesters, polycarbonates, and poly-conjugated dienes each having a structure derived from a C4 to C18 aliphatic diol unit having a branched structure. A polyol having the polymer skeleton mentioned above can be used for the production of urethanized (meth)acrylic compound (b-3a).

Examples of the compound having an isocyanate group include hexamethylene diisocyanate (HDI), tolylene diisocyanate (TDI), xylylene diisocyanate (XDI), diphenylmethane diisocyanate (MDI), isophorone diisocyanate (IPDI), trimethylhexamethylene diisocyanate (TMHMDI), tricyclodecane diisocyanate (TCDDI), and adamantane diisocyanate (ADI).

Examples of the (meth)acrylic compound having a hydroxyl group include:
hydroxy (meth)acrylate compounds such as 2-hydroxyethyl (meth)acrylate, 2-hydroxypropyl (meth)acrylate, 2-hydroxybutyl (meth)acrylate, 6-hydroxyhexyl (meth)acrylate, 10-hydroxydecyl (meth)acrylate, 3-chloro-2-hydroxypropyl (meth)acrylate, 2-hydroxy-3-phenoxypropyl (meth)acrylate, glycerin mono(meth)acrylate, 2-hydroxy-3-acryloyloxypropyl (meth)acrylate, 2,2-bis[4-[3-(meth)acryloyloxy-2-hydroxypropoxy]phenyl]propane, 1,2-bis[3-(meth)acryloyloxy-2-hydroxypropoxy]ethane, pentaerythritol tri(meth)acrylate, and tri or tetra(meth)acrylate of dipentaerythritol; and
hydroxy (meth)acrylamide compounds such as N-hydroxyethyl(meth)acrylamide, and N,N-bis(2-hydroxyethyl)(meth)acrylamide.

Examples of the C4 to C18 aliphatic diol having a branched structure include 2-methyl-1,3-propanediol, 2,2-diethyl-1,3-propanediol, 1,3-butanediol, 2-methyl-1,4-butanediol, neopentyl glycol, 3-methyl-1,5-pentanediol, 2-methyl-1,8-octanediol, 2,7-dimethyl-1,8-octanediol, 2-methyl-1,9-nonanediol, 2,8-dimethyl-1,9-nonanediol, 2-methyl-1,10-decanediol, 2,9-dimethyl-1,10-decanediol, 2-methyl-1,11-undecanediol, 2,10-dimethyl-1,11-undecanediol, 2-methyl-1,12-dodecanediol, 2,11-dimethyl-1,12-dodecanediol, 2-methyl-1,13-tridecanediol, 2,12-dimethyl-1,13-tridecanediol, 2-methyl-1,14-tetradecanediol, 2,13-dimethyl-1,14-tetradecanediol, 2-methyl-1,15-pentadecanediol, 2,14-dimethyl-1,15-pentadecanediol, 2-methyl-1,16-hexadecanediol, and 2,15-dimethyl-1,16-hexadecanediol. In view of providing a dental composition having superior curability, the polyol components used are preferably C5 to C12 aliphatic diols having a methyl-group side chain, such as 2-methyl-1,4-butanediol, 3-methyl-1,5-pentanediol, 2-methyl-1,8-octanediol, 2,7-dimethyl-1,8-octanediol, 2-methyl-1,9-nonanediol, and 2,8-dimethyl-1,9-nonanediol. The polyol components are more preferably 2-methyl-1,4-butanediol, 3-methyl-1,5-pentanediol, 2-methyl-1,8-octanediol, and 2,7-dimethyl-1,8-octanediol, even more preferably 3-methyl-1,5-pentanediol, and 2-methyl-1,8-octanediol.

The addition reaction of the compound having an isocyanate group and the (meth)acrylic compound having a hydroxyl group can be performed following a known method, and the method is not particularly limited.

The urethanized (meth)acrylic compound (b-3a) obtained is, for example, a product of a reaction of any combination of: the polyol having at least one structure selected from the group consisting of a polyester, a polycarbonate, a polyurethane, a polyether, a poly-conjugated diene, and a hydrogenated poly-conjugated diene; the compound having an isocyanate group; and the (meth)acrylic compound having a hydroxyl group.

### · (Meth)Acrylic Compound (b-3b) Having no Urethane Skeleton

The (meth)acrylic compound (b-3b) having no urethane skeleton preferably has a structure (a polymer skeleton) selected from the group consisting of a polyester, a polycarbonate, a polyurethane, a polyether, a poly-conjugated diene, and a hydrogenated poly-conjugated diene.

In such structures, examples of the polyester include a copolymer of a dicarboxylic acid (e.g., an aromatic dicarboxylic acid such as phthalic acid or isophthalic acid, or an unsaturated aliphatic dicarboxylic acid such as maleic acid) and an aliphatic diol having 2 to 18 carbon atoms; a copolymer of a dicarboxylic acid (e.g., a saturated aliphatic dicarboxylic acid such as adipic acid or sebacic acid) and an aliphatic diol having 2 to 18 carbon atoms; a polymer of β-propiolactone; a polymer of γ-butyrolactone; a polymer of δ-valerolactone; a polymer of ε-caprolactone; and a copolymer of these. Preferred are a copolymer of a dicarboxylic acid (an aromatic dicarboxylic acid such as phthalic acid or isophthalic acid, or an unsaturated aliphatic dicarboxylic acid such as maleic acid) and an aliphatic diol having 2 to 12 carbon atoms; and a copolymer of a dicarboxylic acid (a saturated aliphatic dicarboxylic acid such as adipic acid or sebacic acid) and an aliphatic diol having 2 to 12 carbon atoms.

Examples of the polycarbonate include polycarbonates derived from C2 to C18 aliphatic diols, polycarbonates derived from bisphenol A, and polycarbonates derived from C2 to C18 aliphatic diols and bisphenol A. Preferred are polycarbonates derived from C2 to C12 aliphatic diols, polycarbonates derived from bisphenol A, and polycarbonates derived from C2 to C12 aliphatic diols and bisphenol A.

Examples of the polyurethane include polymers of C2 to C18 aliphatic diols and C1 to C18 diisocyanates. Preferred are polymers of C2 to C12 aliphatic diols and C1 to C12 diisocyanates.

Examples of the polyether include polyethylene glycol, polypropylene glycol, polybutylene glycol, and poly(1-methylbutylene glycol).

Examples of the poly-conjugated diene and hydrogenated poly-conjugated diene include 1,4-polybutadiene, 1,2-polybutadiene, polyisoprene, poly(butadiene-isoprene), poly(butadiene-styrene), poly(isoprene-styrene), polyfarnesene, and hydrogenated products of these. In view of superior flexibility and superior water resistance, preferred among these are the polyester, polycarbonate, and poly-conjugated diene structures. A polyol having the polymer skeleton mentioned above can be used for the production of (meth)acrylic compound (b-3b) having no urethane skeleton.

The glass transition temperature and acetone solubility of the (meth)acrylic compound (b-3b) having no urethane skeleton can be adjusted by adjusting the skeleton or molecular weight of a structure (polymer skeleton) selected from the group consisting of a polyester, a polycarbonate, a polyurethane, a polyether, a poly-conjugated diene, and a hydrogenated poly-conjugated diene.

The (meth)acrylic compound (b-3b) having no urethane skeleton obtained is, for example, a product of a reaction of any combination of: the polyol having at least one structure selected from the group consisting of a polyester, a polycarbonate, a polyurethane, a polyether, a poly-conjugated diene, and a hydrogenated poly-conjugated diene; and the (meth)acrylic compound having a hydroxyl group.

Examples of the polyester, polycarbonate, polyurethane, polyether, poly-conjugated diene, hydrogenated poly-conjugated diene, and (meth)acrylic compound having a hydroxyl group include those exemplified for the urethanized (meth)acrylic compound (b-3a).

In view of the effect to reduce viscosity and polymerization shrinkage stress, the (meth)acrylic compound (b-3) has a weight-average molecular weight (Mw) of preferably 1,000 to 80,000, more preferably 2,000 to 50,000, even more preferably 3,000 to 20,000.

In the present invention, weight-average molecular weight (Mw) refers to a weight-average molecular weight measured by gel permeation chromatography (GPC) in terms of polystyrene.

The (meth)acrylic compound (b-3) has a weight-average molecular weight of preferably 1,250 or more and less than 20,000, more preferably 1,500 or more and 17,500 or less, even more preferably 1,800 or more and 16,000 or less, particularly preferably 2,500 or more and 15,000 or less per (meth)acryl group. When the relationship between the number of polymerizable groups and the weight-average molecular weight of the (meth)acrylic compound (b-3) falls within the foregoing ranges, crosslinkage takes place in a moderate fashion, and it is possible to more effectively maintain the mechanical strength while reducing polymerization shrinkage stress.

When the (meth)acrylic compound (b-3) contains polymerizable groups other than (meth)acryl groups, for example, such as vinyl groups or styrene groups, the number of such polymerizable groups other than (meth)acryl groups in the (meth)acrylic compound (b-3) is preferably 2 or less, more preferably 0, because the presence of such polymerizable groups may lead to increased polymerization shrinkage stress depending on the form of polymerization.

In certain embodiments, the polarity difference between (meth)acrylic compound (b-3) and other monomer components is important for obtaining an even greater polymerization shrinkage stress reducing effect, and, to this end, it is preferable for the (meth)acrylic compound (b-3) to have a lower solubility in acetone relative to other monomers having a higher solubility in acetone. In view of the compatibility with other monomer components, the (meth)acrylic compound (b-3) has a solubility in acetone of preferably less than 50 g/L, more preferably less than 30 g/L, even more preferably less than 25 g/L, particularly preferably less than 20 g/L, most preferably less than 10 g/L.

In view of handling properties and the polymerization shrinkage stress reducing effect, the (meth)acrylic compound (b-3) has a viscosity at 25°C of preferably 1,000 to 10,000,000 mPa·s, more preferably 5,000 to 7,500,000 mPa·s, even more preferably 10,000 to 7,000,000 mPa·s. The viscosity means a viscosity measured at 25°C with a Brookfield rotary viscometer. Measurement conditions, such as time and rotational speed, are appropriately adjusted according to the viscosity range.

The (meth)acrylic compound (b-3) may be a commercially available product.

Examples of such commercially available products include urethane polymers having a terminal polymerizable group, such as the Art Resin series (UN7600, UN7700) manufactured by Negami Chemical Industrial Co., Ltd.; the Kuraprene series (polyisoprene skeleton or polybutadiene skeleton) manufactured by Kuraray Co., Ltd. (LIR-30, LIR-50, LIR-390, LIR-403, LIR-410, UC-102M, UC-203M, LIR-700, LBR-302, LBR-307, LBR-305, LBR-352, LBR-361, L-SBR-820, L-SBR-841); polyols manufactured by Kuraray Co., Ltd. (P-6010, P-5010, P-4010, P-3010, P-2010, P-1010, F-3010, F2010, F-1010, P-2011, P-1020, P-2020, P-530, P-2030, P-2050, C-2090); and the liquid polybutadiene NISSO-PB manufactured by Nippon Soda Co., Ltd. (B-1000, B-2000, B-3000, BI-2000, BI-3000, G-1000, G-2000, G-3000, GI-1000, GI-2000, GI-3000, TEAI-1000, TE-2000, TE-4000, JP-100, JP-200). Preferred are UN7600, UN7700, LBR-302, LBR-307, LBR-305, LBR-352, LBR-361, L-SBR-820, UC-102M, UC-203M, C-2090, P-2020, P-2050, B3000, BI-2000, BI-3000, TEAI-1000, TE-2000, and TE-4000. More preferred are UN7600, UN7700, UC-102M, TE-2000, and TE-4000.

In view of mechanical strength, paste properties, and polymerization shrinkage stress reducing effect, the content of the (meth)acrylic compound (b-3) in a dental composition of the present invention is preferably 0.1 to 50 parts by mass, more preferably 0.5 to 40 parts by mass, even more preferably 1 to 35 parts by mass, particularly preferably 5 to 25 parts by mass in total 100 parts by mass of the monomers.

In certain embodiments, in view of mechanical strength, paste properties, and polymerization shrinkage stress reducing effect, the content of the (meth)acrylic compound (b-3) is preferably 0.1 mass% or more, more preferably 0.5 mass% or more, even more preferably 1 mass% or more, particularly preferably 1.5 mass% or more in total 100 mass% of the dental composition.

In view of mechanical strength, paste properties, and polymerization shrinkage stress reducing effect, the content of the (meth)acrylic compound (b-3) is preferably 30 mass% or less, more preferably 25 mass% or less, even more preferably 20 mass% or less, particularly preferably 15 mass% or less in total 100 mass% of the dental composition.

In view of the properties of the dental composition, adhesive properties to tooth structure, and mechanical strength, the total content of the monomer (b) having no acidic group in the dental composition is preferably 60 to 99 parts by mass, more preferably 65 to 97.5 parts by mass, even more preferably 70 to 95 parts by mass, particularly preferably 75 to 95 parts by mass in total 100 parts by mass of the monomers.

In view of adhesive properties to tooth structure, the mass ratio of hydrophobic monomer (b-1) to hydrophilic monomer (b-2) is preferably 1:0 to 1:2, more preferably 1:0 to 1:1, even more preferably 1:0 to 2:1.

### <Polymerization Initiator (c)>

A dental composition of the present invention comprises a polymerization initiator (c) to cure the monomers.

The polymerization initiator (c) may be a photopolymerization initiator (c-1) or chemical polymerization initiator (c-2). The photopolymerization initiator (c-1) or chemical polymerization initiator (c-2) may be used alone, or two or more thereof may be used in combination.

The photopolymerization initiator (c-1) can be classified into water-soluble photopolymerization initiator (c-1a) and water-insoluble photopolymerization initiator (c-1b).

The photopolymerization initiator (c-1) may be solely a water-soluble photopolymerization initiator (c-1a) or a water-insoluble photopolymerization initiator (c-1b), or may be a combination of a water-soluble photopolymerization initiator (c-1a) and a water-insoluble photopolymerization initiator (c-1b). Preferably, a water-soluble photopolymerization initiator (c-1a) and a water-insoluble photopolymerization initiator (c-1b) are used in combination.

### · Water-Soluble Photopolymerization Initiator (c-1a)

The water-soluble photopolymerization initiator (c-1a) can improve the polymerization curability at the interface with hydrophilic tooth surfaces, resulting in high adhesive strength.

The water-soluble photopolymerization initiator (c-1a) has a solubility in 25°C water of 10 g/L or more, preferably 15 g/L or more, more preferably 20 g/L or more, even more preferably 25 g/L or more.

By having a solubility of 10 g/L or more in 25°C water, the water-soluble photopolymerization initiator (c-1a) can sufficiently dissolve in water within the tooth structure at the adhesive interface, and more easily exhibits the polymerization promoting effect.

Examples of the water-soluble photopolymerization initiator (c-1a) include water-soluble thioxanthones; water-soluble acylphosphine oxides; and α-hydroxyalkylacetophenones, for example, such as compounds having a (poly)ethylene glycol chain introduced into the hydroxyl group(s) of 1-[4-(2-hydroxyethoxy)-phenyl]-2-hydroxy-2-methyl-1-propan-1-one, compounds having a (poly)ethylene glycol chain introduced into the hydroxyl group and/or phenyl group of 1-hydroxycyclohexyl phenyl ketone, compounds having -OCH₂COO⁻Na⁺ introduced into the phenyl group of 1-hydroxycyclohexyl phenyl ketone, compounds having a (poly)ethylene glycol chain introduced into the hydroxyl group and/or phenyl group of 2-hydroxy-2-methyl-1-phenylpropan-1-one, and compounds having -OCH₂COO⁻Na⁺ introduced into the phenyl group of 2-hydroxy-2-methyl-1-phenylpropan-1-one. Other examples of the water-soluble photopolymerization initiator (c-1a) include quaternary ammonium compounds prepared by quaternization of the amino group of α-aminoalkylphenones, such as 2-methyl-1-[4-(methylthio)phenyl]-2-morpholinopropan-1-one, and 2-benzyl-2-(dimethylamino)-1-[(4-morpholino)phenyl]-1-butanon.

The water-soluble thioxanthones may be any of, for example, 2-hydroxy-3-(9-oxo-9H-thioxanthen-4-yloxy)-N,N,N-trimethyl-1-propaneaminium chloride, 2-hydroxy-3-(1-methyl-9-oxo-9H-thioxanthen-4-yloxy)-N,N,N-trimethyl-1-propaneaminium chloride, 2-hydroxy-3-(9-oxo-9H-thioxanthen-2-yloxy)-N,N,N-trimethyl-1-propaneaminium chloride, 2-hydroxy-3-(3,4-dimethyl-9-oxo-9H-thioxanthen-2-yloxy)-N,N,N-trimethyl-1-propaneaminium chloride, 2-hydroxy-3-(3,4-dimethyl-9H-thioxanthen-2-yloxy)-N,N,N-trimethyl-1-propaneaminium chloride, and 2-hydroxy-3-(1,3,4-trimethyl-9-oxo-9H-thioxanthen-2-yloxy)-N,N,N-trimethyl-1-propaneaminium chloride.

Examples of the water-soluble acylphosphine oxides include acylphosphine oxides represented by the following general formula (1), (2), or (3).

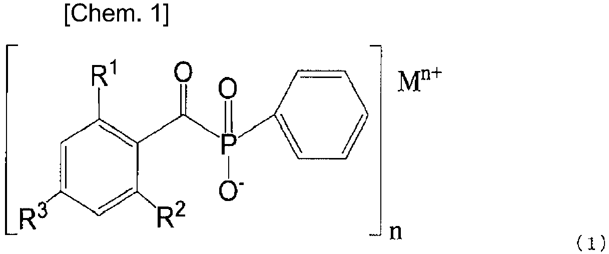

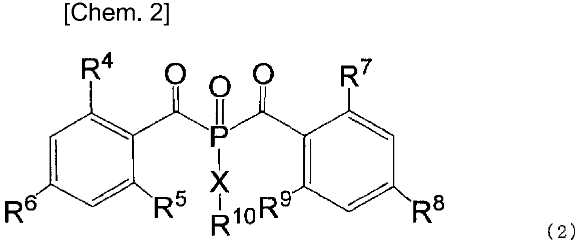

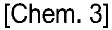

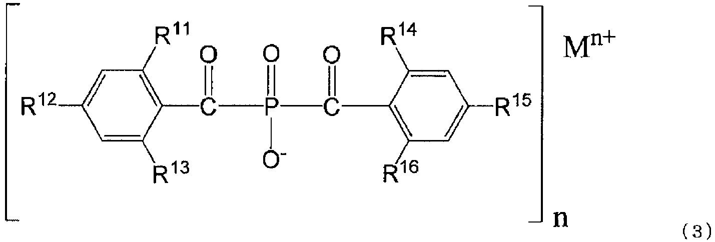

In formulae (1), (2), and (3), R¹ to R⁹ and R¹¹ to R¹⁶ are each independently a C₁ to C₄ linear or branched alkyl group or a halogen atom, M is a hydrogen ion, an alkali metal ion, an alkali earth metal ion, a magnesium ion, a pyridinium ion (the pyridine ring may have a substituent), or an ammonium ion represented by HN⁺R¹⁷R¹⁸R¹⁹ (where R¹⁷, R¹⁸, and R¹⁹ are each independently an organic group or a hydrogen atom), n is 1 or 2, X is a C₁ to C₄ linear or branched alkylene group, and R¹⁰ represents - CH(CH₃)COO(C₂H₄O)ₚCH₃, where p represents an integer of 1 to 1,000.

The alkyl groups represented by R¹ to R⁹ and R¹¹ to R¹⁶ are not particularly limited, as long as these are C₁ to C₄ linear or branched alkyl groups. Examples include methyl groups, ethyl groups, n-propyl groups, isopropyl groups, n-butyl groups, isobutyl groups, sec-butyl groups, 2-methylpropyl groups, and tert-butyl groups.

The alkyl groups represented by R¹ to R⁹ and R¹¹ to R¹⁶ are preferably C₁ to C₃ linear alkyl groups, more preferably methyl or ethyl groups, even more preferably methyl groups.

Examples of X include methylene groups, ethylene groups, n-propylene groups, isopropylene groups, and n-butylene groups. X is preferably a C₁ to C₃ linear alkylene group, more preferably a methylene or ethylene group, even more preferably a methylene group.

Examples of the substituent of the pyridine ring when M is a pyridinium ion include halogen atoms (a fluorine atom, a chlorine atom, a bromine atom, an iodine atom), carboxyl groups, C₂ to C₆ linear or branched acyl groups, C₁ to C₆ linear or branched alkyl groups, and C₁ to C₆ linear or branched alkoxy groups. Preferably, M is an alkali metal ion, an alkali earth metal ion, a magnesium ion, a pyridinium ion (the pyridine ring may have a substituent), or an ammonium ion represented by HN⁺R¹⁷R¹⁸R¹⁹ (the symbols have the same meaning as above). Examples of the alkali metal ion include a lithium ion, a sodium ion, a potassium ion, a rubidium ion, and a cesium ion. Examples of the alkali earth metal ion include a calcium ion, a strontium ion, a barium ion, and a radium ion.

The organic groups represented by R¹⁷, R¹⁸, and R¹⁹ may be the same groups exemplified above for the substituent of the pyridine ring (excluding a halogen atom).

In view of adhesive properties to tooth structure, particularly preferred are compounds in which R¹ to R³ are all methyl groups in general formula (1), compounds in which R⁴ to R⁹ are all methyl groups in general formula (2), and compounds in which R¹¹ to R¹⁶ are all methyl groups in general formula (3). Examples of ammonium ions include ammonium ions derived from various amines. Examples of the amines include ammonia, trimethylamine, diethylamine, dimethylaniline, ethylenediamine, triethanolamine, N,N-dimethylaminomethacrylate, 4-(N,N-dimethylamino)benzoic acid and alkyl esters thereof, 4-(N,N-diethylamino)benzoic acid and alkyl esters thereof, and N,N-bis(2-hydroxyethyl)-p-toluidine.

In view of adhesive properties, p in R¹⁰ is 1 or more, preferably 2 or more, more preferably 3 or more, even more preferably 4 or more. In view of adhesive properties, p in R¹⁰ is 1,000 or less, preferably 100 or less, more preferably 75 or less, even more preferably 50 or less.

Particularly preferred among these water-soluble acylphosphine oxides are sodium phenyl(2,4,6-trimethylbenzoyl)phosphinate, lithium phenyl(2,4,6-trimethylbenzoyl)phosphinate, sodium bis(2,4,6-trimethylbenzoyl)phosphinate, lithium bis(2,4,6-trimethylbenzoyl)phosphinate, and compounds represented by general formula (2) and in which the moiety corresponding to the group represented by R¹⁰ is synthesized from polyethylene glycol methyl ether methacrylate having a molecular weight of 950.

The water-soluble acylphosphine oxides having such structures can be synthesized according to known methods, and some are available as commercially available products. For example, the methods disclosed in JP S57-197289 A and WO2014/095724 can be used for the synthesis of the water-soluble acylphosphine oxides.

The water-soluble photopolymerization initiator (c-1a) may be used alone, or two or more thereof may be used in combination.

The water-soluble photopolymerization initiator (c-1a) may be dissolved in the dental composition, or may be dispersed in powder form within the dental composition.

When the water-soluble photopolymerization initiator (c-1a) is dispersed in powder form within the dental composition, the average particle diameter of the powder is preferably 500 µm or less, more preferably 100 µm or less, even more preferably 50 µm or less because the water-soluble photopolymerization initiator (c-1a) tends to settle when the powder has an excessively large average particle diameter. The average particle diameter is preferably 0.01 µm or more because the specific surface area of the powder overly increases, and the amount of powder that can be dispersed within the dental composition decreases when the powder has an excessively small average particle diameter. Taken together, the average particle diameter of water-soluble photopolymerization initiator (c-1a) preferably ranges from 0.01 to 500 µm, more preferably 0.01 to 100 µm, even more preferably 0.01 to 50 µm.

The average particle diameter of a powder of individual water-soluble photopolymerization initiators (c-1a) can be determined by taking an electron micrograph of at least 100 particles, and calculating the volume average particle diameter from the captured image after an image analysis with image-analyzing particle size distribution measurement software (Mac-View, manufactured by Mountech Co., Ltd.).

The shape of the water-soluble photopolymerization initiator (c-1a) when it is dispersed in powder form is not particularly limited, and may be any of various shapes, including, for example, spherical, stylus, plate-like, and crushed shapes. The water-soluble photopolymerization initiator (c-1a) can be prepared using a known method such as pulverization, freeze drying, or reprecipitation. In view of the average particle diameter of the powder obtained, freeze drying and reprecipitation are preferred, and freeze drying is more preferred.

In view of curability and other properties of the dental composition obtained, the content of water-soluble photopolymerization initiator (c-1a) is preferably 0.01 to 20 parts by mass relative to total 100 parts by mass of monomers in the dental composition. In view of greater adhesive properties to tooth structure, the content of water-soluble photopolymerization initiator (c-1a) is more preferably 0.05 to 10 parts by mass, even more preferably 0.1 to 5 parts by mass relative to total 100 parts by mass of monomers in the dental composition.

When the content of water-soluble photopolymerization initiator (c-1a) is at or above these lower limits, polymerization can sufficiently proceed at the adhesive interface, making it easier to provide a sufficient adhesive strength. When the content of water-soluble photopolymerization initiator (c-1a) is at or below the foregoing upper limits, it is easier to provide a sufficient adhesive strength.

### · Water-Insoluble Photopolymerization Initiator (c-1b)

Preferably, the dental composition comprises a water-insoluble photopolymerization initiator (c-1b) having a solubility of less than 10 g/L in 25°C water (hereinafter, also referred to as "water-insoluble photopolymerization initiator (c-1b)"). The dental composition may comprise the water-insoluble photopolymerization initiator (c-1b) alone. However, in view of curability and mechanical strength, it is preferable that the dental composition additionally comprises a water-insoluble photopolymerization initiator (c-1b) having a solubility of less than 10 g/L in 25°C water, together with the water-soluble photopolymerization initiator (c-1a).

The water-insoluble photopolymerization initiator (c-1b) used in the present invention may be a known photopolymerization initiator. The water-insoluble photopolymerization initiator (c-1b) may be incorporated alone, or two or more thereof may be incorporated in combination.

Examples of the water-insoluble photopolymerization initiator (c-1b) include (bis)acylphosphine oxides (other than those exemplified for the water-soluble photopolymerization initiator (c-1a)), thioxanthones, ketals, α-diketones, coumarins, anthraquinones, benzoin alkyl ether compounds, and α-aminoketone compounds.

Examples of acylphosphine oxides in the (bis)acylphosphine oxides include 2,4,6-trimethylbenzoyldiphenylphosphine oxide, 2,6-dimethoxybenzoyldiphenylphosphine oxide, 2,6-dichlorobenzoyldiphenylphosphine oxide, 2,4,6-trimethylbenzoylmethoxyphenylphosphine oxide, 2,4,6-trimethylbenzoylethoxyphenylphosphine oxide, 2,3,5,6-tetramethylbenzoyldiphenylphosphine oxide, and benzoyl di(2,6-dimethylphenyl)phosphonate. Examples of bisacylphosphine oxides include bis(2,6-dichlorobenzoyl)phenylphosphine oxide, bis(2,6-dichlorobenzoyl)-2,5-dimethylphenylphosphine oxide, bis(2,6-dichlorobenzoyl)-4-propylphenylphosphine oxide, bis(2,6-dichlorobenzoyl)-1-naphthylphosphine oxide, bis(2,6-dimethoxybenzoyl)phenylphosphine oxide, bis(2,6-dimethoxybenzoyl)-2,4,4-trimethylpentylphosphine oxide, bis(2,6-dimethoxybenzoyl)-2,5-dimethylphenylphosphine oxide, bis(2,4,6-trimethylbenzoyl)phenylphosphine oxide, and bis(2,5,6-trimethylbenzoyl)-2,4,4-trimethylpentylphosphine oxide.

Examples of the thioxanthones include thioxanthone, and 2-chlorothioxanthen-9-one.

Examples of the ketals include benzyl dimethyl ketal, and benzyl diethyl ketal.

Examples of the α-diketones include diacetyl, benzyl, dl-camphorquinone, 2,3-pentadione, 2,3-octadione, 9,10-phenanthrenequinone, 4,4'-oxybenzyl, and acenaphthenequinone. Particularly preferred is dl-camphorquinone for its maximum absorption wavelength occurring in the visible light region.

Examples of the coumarins include compounds mentioned in JP H09-3109 A and JP H10-245525 A, including, for example, 3,3'-carbonylbis(7-diethylaminocoumarin), 3-(4-methoxybenzoyl)coumarin, 3-thienoylcoumarin, 3-benzoyl-5,7-dimethoxycoumarin, 3-benzoyl-7-methoxycoumarin, 3-benzoyl-6-methoxycoumarin, 3-benzoyl-8-methoxycoumarin, 3-benzoylcoumarin, 7-methoxy-3-(p-nitrobenzoyl)coumarin, 3-(p-nitrobenzoyl)coumarin, 3,5-carbonylbis(7-methoxycoumarin), 3-benzoyl-6-bromocoumarin, 3,3'-carbonylbiscoumarin, 3-benzoyl-7-dimethylaminocoumarin, 3-benzoylbenzo[f]coumarin, 3-carboxycoumarin, 3-carboxy-7-methoxycoumarin, 3-ethoxycarbonyl-6-methoxycoumarin, 3-ethoxycarbonyl-8-methoxycoumarin, 3-acetylbenzo[f]coumarin, 3-benzoyl-6-nitrocoumarin, 3-benzoyl-7-diethylaminocoumarin, 7-dimethylamino-3-(4-methoxybenzoyl)coumarin, 7-diethylamino-3-(4-methoxybenzoyl)coumarin, 7-diethylamino-3-(4-diethylamino)coumarin, 7-methoxy-3-(4-methoxybenzoyl)coumarin, 3-(4-nitrobenzoyl)benzo[f]coumarin, 3-(4-ethoxycinnamoyl)-7-methoxycoumarin, 3-(4-dimethylaminocinnamoyl)coumarin, 3-(4-diphenylaminocinnamoyl)coumarin, 3-[(3-dimethylbenzothiazol-2-ylidene)acetyl]coumarin, 3-[(1-methylnaphtho[1,2-d]thiazol-2-ylidene)acetyl]coumarin, 3,3'-carbonylbis(6-methoxycoumarin), 3,3'-carbonylbis(7-acetoxycoumarin), 3,3'-carbonylbis(7-dimethylaminocoumarin), 3-(2-benzothiazolyl)-7-(diethylamino)coumarin, 3-(2-benzothiazolyl)-7-(dibutylamino)coumarin, 3-(2-benzoimidazolyl)-7-(diethylamino)coumarin, 3-(2-benzothiazolyl)-7-(dioctylamino)coumarin, 3-acetyl-7-(dimethylamino)coumarin, 3,3'-carbonylbis(7-dibutylaminocoumarin), 3,3'-carbonyl-7-diethylaminocoumarin-7'-bis(butoxyethyl)aminocoumarin, 10-[3-[4-(dimethylamino)phenyl]-1-oxo-2-propenyl]-2,3,6,7-tetrahydro-1,1,7,7-tetramethyl-1H,5H,11H-[1]benzopyrrano[6,7,8-ij]quinolizin-11-one, and 10-(2-benzothiazolyl)-2,3,6,7-tetrahydro-1,1,7,7-tetramethyl-1H,5H,11H-[1]benzopyrrano[6,7,8-ij]quinolizin-11-one.

Particularly preferred among these coumarins are 3,3'-carbonylbis(7-diethylaminocoumarin) and 3,3'-carbonylbis(7-dibutylaminocoumarin).

Examples of the anthraquinones include anthraquinone, 1-chloroanthraquinone, 2-chloroanthraquinone, 1-bromoanthraquinone, 1,2-benzanthraquinone, 1-methylanthraquinone, 2-ethylanthraquinone, and 1-hydroxyanthraquinone.

Examples of the benzoin alkyl ether compounds include benzoin methyl ether, benzoin ethyl ether, benzoin isopropyl ether, and benzoin isobutyl ether.

Examples of the α-aminoketone compounds include 2-methyl-1-[4-(methylthio)phenyl]-2-morpholinopropan-1-one.

Among these examples, the water-insoluble photopolymerization initiator (c-1b) is preferably at least one selected from the group consisting of a (bis)acylphosphine oxide, an α-diketone, and a coumarin. In this way, a dental composition can be obtained that has excellent photocurability both in the visible light region and the near ultraviolet region, and that shows sufficient photocurability regardless of whether the light source used is a halogen lamp, a light emitting diode (LED), or a xenon lamp.

The content of water-insoluble photopolymerization initiator (c-1b) is not particularly limited. However, in view of curability and other properties of the dental composition obtained, the content of water-insoluble photopolymerization initiator (c-1b) is preferably 0.01 to 10 parts by mass, more preferably 0.05 to 7 parts by mass, even more preferably 0.1 to 5 parts by mass relative to total 100 parts by mass of monomers in the dental composition.

When the content of water-insoluble photopolymerization initiator (c-1b) is at or below these upper limits, it is easier to obtain a sufficient adhesive strength even when the water-insoluble photopolymerization initiator (c-1b) itself has low polymerization performance, in addition to reducing precipitation of the water-insoluble photopolymerization initiator (c-1b) itself from the dental composition.

When the water-soluble photopolymerization initiator (c-1a) and the water-insoluble photopolymerization initiator (c-1b) are used in combination, the mass ratio (c-1a):(c-1b) of water-soluble photopolymerization initiator (c-1a) and water-insoluble photopolymerization initiator (c-1b) in the present invention is preferably 10:1 to 1:10, more preferably 7:1 to 1:7, even more preferably 5:1 to 1:5, particularly preferably 3:1 to 1:3.

When the fraction of water-soluble photopolymerization initiator (c-1a) in the mass ratio exceeds 10:1, it may lead to a decrease in adhesive strength to tooth structure or flexural strength due to a decrease in the curability of the dental composition itself, potentially presenting difficulties in achieving the effects of the present invention.

When the fraction of water-insoluble photopolymerization initiator (c-1b) in the mass ratio exceeds 1:10, the dental composition may have difficulty in exhibiting high adhesive strength as a result of insufficient promotion of polymerization at the adhesive interface, though the curability of the dental composition itself can increase.

### · Chemical Polymerization Initiator (c-2)

In view of enabling chemical polymerization, the dental composition may additionally comprise a chemical polymerization initiator (c-2). Preferred for use as chemical polymerization initiator (c-2) are organic peroxides. The organic peroxides used as chemical polymerization initiator (c-2) are not particularly limited, and known organic peroxides may be used. Typical examples of such organic peroxides include ketone peroxides, hydroperoxides, diacyl peroxides, dialkyl peroxides, peroxyketals, peroxyesters, and peroxydicarbonates. Specific examples of these organic peroxides include those mentioned in WO2008/087977. The chemical polymerization initiator may be used alone, or two or more thereof may be used in combination.

### <Filler (d)>

The dental composition comprises a filler (d) to adjust handling properties, while also aiming to enhance the mechanical strength (such as flexural strength) of the cured product, and to increase the adhesive properties to tooth structure.

Examples of filler (d) include inorganic fillers, organic-inorganic composite fillers, and organic fillers. The filler (d) may be used alone, or two or more thereof may be used in combination.

The material of inorganic fillers preferably contains various types of glass (primarily composed of silica (a silica content of 5 mass% or more, preferably 10 mass% or more), with optional oxides of elements such as heavy metals, boron, and aluminum).

Examples of the inorganic fillers include glass powders of common compositions, such as fused silica, quartz, soda-lime-silica glass, E glass, C glass, and borosilicate glass (PYREX^{®} glass). Other examples include composite oxides (such as barium glass, strontium borosilicate glass, lanthanum glass-ceramics, fluoroaluminosilicate glass, various ceramics, alumina, silica-titania, silica-zirconia, ytterbium oxide, silica-coated ytterbium fluoride, aluminosilicate glass, barium boroaluminosilicate glass, calcium fluoroaluminosilicate glass, strontium fluoroaluminosilicate glass, barium fluoroaluminosilicate glass, and strontium calcium fluoroaluminosilicate glass), diatomaceous earth, kaolin, clay minerals (such as montmorillonite), activated earth, synthetic zeolite, mica, calcium fluoride, ytterbium fluoride, yttrium fluoride, calcium phosphate, barium sulfate, zirconium oxide, titanium oxide, and hydroxyapatite. These may be used alone, or two or more thereof may be used in combination.

Preferred among these are inorganic fillers composed of metallic elements that provide high X-ray contrast properties, such as aluminum, strontium, zirconium, barium, lanthanum, ytterbium, titanium, and bismuth (for example, inorganic fillers such as barium glass, alumina, silica-titania, silica-zirconia, and silica-coated ytterbium fluoride).

The inorganic fillers may be used as inorganic fillers with a relatively high refractive index. These may be used alone, or two or more thereof may be used as a mixture.

In view of providing superior mechanical strength and transparency in the cured product obtained, preferred are quartz, silica, silica-titania, silica-zirconia, barium glass, ytterbium oxide, and silica-coated ytterbium fluoride, more preferably quartz, silica, silica-titania, silica-zirconia, barium glass, and silica-coated ytterbium fluoride.

In view of the radiopacity of the dental composition, preferred are silica-zirconia, barium glass, and silica-coated ytterbium fluoride, particularly preferably silica-zirconia, and barium glass.

The inorganic fillers may be commercially available products.

Examples of such commercially available products include silica such as Aerosil^{®} 90, Aerosil^{®} 130, Aerosil^{®} 150, Aerosil^{®} 200, Aerosil^{®} 255, Aerosil^{®} 300, Aerosil^{®} 380, Aerosil^{®} OX50, and Aerosil^{®} R972 (all manufactured by Nippon Aerosil Co., Ltd.), barium glass such as GM27884, 8235 (manufactured by Schott), and E-3000 (product code; manufactured by Esstech), strontium borosilicate glass (E-4000, manufactured by ESSTECH), lanthanum glass-ceramics (GM31684, manufactured by Schott), and fluoroaluminosilicate glass (GM35429, G018-091, and G018-117, manufactured by Schott).

The inorganic fillers may be amorphous or crystalline, or a combination of these. However, the inorganic fillers are preferably at least partially amorphous.

The shape of inorganic fillers is not particularly limited, and the particle diameter of fillers may be appropriately selected for use. Examples include irregularly shaped fillers (crushed fillers) and spherical fillers. In view of improving the mechanical strength of the cured product of the dental composition, preferred for use as inorganic fillers are irregularly shaped fillers. Here, the spherical fillers are particles that appear round in shape when observed in a unit field of an electron micrograph, and that have an average uniformity of 0.6 or more as calculated by dividing the diameter of a particle perpendicular to its maximum diameter by the maximum diameter.

In order to adjust the mechanical strength of the cured product and flowability, it is preferable to use the inorganic fillers after a surface treatment with a known surface treatment agent such as a silane coupling agent. For example, the hydroxyl groups present on the surface of inorganic fillers may be surface treated with a surface treatment agent to obtain inorganic fillers having its hydroxyl groups surface treated.

Examples of the surface treatment agent include silane coupling agents such as vinyltrimethoxysilane, vinyltriethoxysilane, vinyltrichlorosilane, vinyltri(β-methoxyethoxy)silane, 3-methacryloyloxypropyltrimethoxysilane, 8-methacryloyloxyoctyltrimethoxysilane, 11-methacryloyloxyundecyltrimethoxysilane, γ-glycidoxypropyltrimethoxysilane, γ-mercaptopropyltrimethoxysilane, and γ-aminopropyltriethoxysilane. Preferred are vinyltrimethoxysilane, 3-methacryloyloxypropyltrimethoxysilane, 8-methacryloyloxyoctyltrimethoxysilane, 11-methacryloyloxyundecyltrimethoxysilane, and γ-aminopropyltriethoxysilane.

The surface treatment can be carried out using any known method, including, for example, a method that adds the surface treatment agent by spraying it while vigorously stirring the inorganic filler, a method that disperses or dissolves the inorganic filler and surface treatment agent in a suitable solvent, and removes the solvent, and a method in which the alkoxy group of the surface treatment agent is transformed into a silanol group by hydrolysis with an acid catalyst in an aqueous solution, and the silanol group is allowed to adhere to the inorganic filler surface in the aqueous solution before removal of water. In all of these methods, the reaction between the surface of inorganic filler and the surface treatment agent can proceed to completion and enable a surface treatment by applying heat in a temperature range of typically 50 to 150°C.

The amount of surface treatment is not particularly limited. For example, 0.1 to 40 parts by mass of surface treatment agent may be used relative to 100 parts by mass of inorganic filler before surface treatment.

The organic-inorganic composite fillers are fillers obtained by adding a monomer to the inorganic filler, polymerizing the mixture in paste form, and pulverizing the resulting material.

The organic-inorganic composite filler refers to a filler comprising the inorganic filler and a polymer of a monomer. The organic-inorganic composite filler may be, for example, a filler obtained by mixing Bis-GMA, 3G, and a surface-treated silica filler, and pulverizing the mixture after polymerization. The shape of the organic-inorganic composite filler is not particularly limited, and the particle diameter of fillers may be appropriately selected for use.

The organic-inorganic composite fillers may be used alone, or two or more thereof may be used as a mixture. In view of mechanical strength, the organic-inorganic composite fillers are preferably surface-treated. Examples of surface treatment agents and the preferred varieties of surface treatment agents are no different from those described in conjunction with inorganic fillers.

In view of properties such as the handling properties and mechanical strength of the dental composition obtained, the organic-inorganic composite fillers have an average particle diameter of preferably 0.001 to 50 µm, more preferably 0.001 to 20 µm, even more preferably 0.005 to 15 µm.

Examples of the materials of the organic fillers include polymethylmethacrylate, polyethylmethacrylate, a methyl methacrylate-ethyl methacrylate copolymer, crosslinked polymethylmethacrylate, crosslinked polyethylmethacrylate, polyamides, polyvinyl chloride, polystyrene, chloroprene rubber, nitrile rubber, an ethylene-vinyl acetate copolymer, a styrene-butadiene copolymer, an acrylonitrile-styrene copolymer, and an acrylonitrile-styrene-butadiene copolymer. These may be used alone, or two or more thereof may be used as a mixture.

The shape of organic fillers is not particularly limited, and the particle diameter of fillers may be appropriately selected for use.

In view of considerations such as the handling properties and mechanical strength of the dental composition obtained, the average particle diameter of the organic fillers is preferably 0.001 to 50 µm, more preferably 0.001 to 20 µm, even more preferably 0.005 to 15 µm.

In this specification, the average particle diameter of filler can be determined by a laser diffraction scattering method or by observing particles with an electron microscope. Specifically, a laser diffraction scattering method is more convenient for the measurement of particles having a particle size of 0.1 µm or more, whereas electron microscopy is a more convenient method of particle size measurement for ultrafine particles of less than 0.1 µm. Here, 0.1 µm is a measured value by a laser diffraction scattering method.

In particles such as agglomerated particles formed by primary particles, there are average particle diameters for primary particles and secondary particles. In such cases, the average particle diameter of fillers refers to the average particle diameter of the larger secondary particles, except where it is specifically stated as the average particle diameter of primary particles.

As a specific example of a laser diffraction scattering method, the particle size may be measured by volume using, for example, a laser diffraction particle size distribution analyzer (SALD-2300, manufactured by Shimadzu Corporation) with a 0.2% sodium hexametaphosphate aqueous solution used as dispersion medium.

In electron microscopy, for example, particles may be photographed with an electron microscope (Model S-4000, manufactured by Hitachi), and the size of particles (at least 200 particles) observed in a unit field of the captured image may be measured using image-analyzing particle-size-distribution measurement software (Mac-View, manufactured by Mountech Co., Ltd.). Here, the particle diameter is determined as an arithmetic mean value of the maximum and minimum lengths of particles, and the average particle diameter is calculated from the number of particles and the particle diameter.

The refractive index of filler (d) is not particularly limited, and may be, for example, 1.35 to 2.00. By bringing the refractive index of filler (d) closer to that of non-filler components in the cured product of the dental composition, it is easier to increase transparency in the cured product obtained. From an aesthetic standpoint where the cured product, as a dental restoration material, should be prevented from appearing unnaturally white compared to natural teeth, it is preferable to provide transparency in the cured product through the approximation of refractive indices. In this respect, the refractive index of filler (d) is preferably 1.40 to 1.70, more preferably 1.45 to 1.65, even more preferably 1.50 to 1.60.

The refractive index of substances under measurement (such as filler (d) and colorants) can be measured with an Abbe refractometer.

Including a plurality of fillers with different refractive indices allows the dental composition to exhibit light diffusing properties. In view of light diffusing properties and favorable shade conformity to both enamel and dentin, the refractive index difference between these multiple fillers is preferably 0.02 or more, more preferably 0.03 or more, even more preferably 0.04 or more.

In view of more readily achieving favorable shade conformity to both enamel and dentin of significantly different shades, the refractive index difference between multiple fillers is preferably less than 0.10, more preferably 0.09 or less, even more preferably 0.08 or less.

### · Aggregate Filler (d-1)

In view of adjusting handling properties and providing not only high mechanical strength, excellent ease of polishing, and favorable paste properties but also high adhesive strength to tooth structure, the dental composition comprises an aggregate filler (d-1) in which primary particles are aggregated. Inorganic fillers are examples of such fillers.

The primary particles constituting the aggregate filler (d-1) are metal oxides.

In this specification, aggregate filler (d-1) refers to secondary particles formed by the fusion of primary particles.

The aggregate filler (d-1) may be used alone, or two or more thereof may be used in combination.

The metal oxides exemplified above as inorganic filler materials can be used as the material of the aggregate filler (d-1).

Examples of the metal elements contained in the metal oxides include boron, silicon, aluminum, strontium, zirconium, barium, lanthanum, ytterbium, titanium, and bismuth, preferably silicon, aluminum, strontium, zirconium, and barium, more preferably silicon, aluminum, zirconium, and barium, even more preferably silicon and/or zirconium, particularly preferably silicon and zirconium.

The metal elements may be used alone, or two or more thereof may be used in combination.

In this specification, boron and silicon are regarded as metal elements.

The metal oxides include composite oxides such as silica-titania, and silica-zirconia.

In view of properties such as the handling properties, adhesive properties to tooth structure, ease of polishing, and mechanical strength of the dental composition obtained, the aggregate filler (d-1) (secondary particles) has an average particle diameter of preferably 1 to 20 µm, more preferably 1.5 to 15 µm, even more preferably 2 to 12.5 µm, most preferably 3 to 10 µm.

In the present invention, the average particle diameter of aggregate filler (d-1) means the average particle diameter before surface treatment when aggregate filler (d-1) is subjected to surface treatment as above.

In view of properties such as the ease of polishing of the dental composition obtained, the primary particles constituting the aggregate filler (d-1) have an average particle diameter of preferably 30 to 500 nm, more preferably 40 to 400 nm, even more preferably 50 to 300 nm, particularly preferably 60 to 200 nm.

With the average particle diameter falling in these ranges, the cured product can exhibit excellent ease of polishing by preventing excessive surface irregularities during polishing, such as particle detachment creating large holes in the surface while polishing the surface.

In the present invention, the average particle diameter of the primary particles constituting the aggregate filler (d-1) means the average particle diameter before surface treatment when the primary particles constituting the aggregate filler (d-1) are subjected to surface treatment as above.

Concerning the average particle diameter of primary particles, a filler (commercially available product) with the desired range of average particle diameters can be purchased as the raw material filler. Alternatively, the raw material filler may be pulverized to produce a filler with its primary particles falling within the desired average particle diameter range.

It is also possible to employ methods such as classification to produce a filler with its primary particles falling within the desired average particle diameter range.

In view of properties such as the ease of polishing, adhesive properties to tooth structure, and mechanical strength of the dental composition obtained, the aggregate filler (d-1) has a specific surface area of 5 m²/g or more and 60 m²/g or less, preferably 8 m²/g or more and less than 50 m²/g, more preferably 10 m²/g or more and 40 m²/g or less, even more preferably 12 m²/g or more and 35 m²/g or less, particularly preferably 15 m²/g or more and 30 m²/g or less as measured by a BET method.

With the specific surface area of aggregate filler (d-1) falling in these ranges, it is possible to achieve excellent ease of polishing while ensuring high mechanical strength in the cured product, as well as favorable paste properties, without compromising the high adhesive strength to tooth structure exhibited by the monomer (a) having an acidic group.

It is to be noted that the specific surface area of aggregate filler (d-1) refers to the specific surface area of the secondary particles.

There is no limitation to the method of production of aggregate filler (d-1), and known methods can be employed.

In an example method for the production of aggregate filler (d-1), the primary particles of the raw material metal oxides are pulverized, if necessary, to the desired average particle diameter, and dispersed in a solvent with an added binder to prepare a slurry. The slurry is subsequently granulated by spray drying. The resulting particles can then be heat-treated to yield an aggregate filler (d-1) having the desired specific surface area.

The method employed for the dispersion process is not particularly limited, and may be a known method, for example, such as ultrasonic dispersion.

The heat treatment is not particularly limited, as long as it enables adjustments to achieve the desired specific surface area, and allows the particles to fuse. However, the heat treatment is conducted at preferably 200°C or more and 1,000°C or less, more preferably 300°C or more and 950°C or less, even more preferably 350°C or more and 900°C or less.

The heat treatment may be followed by a surface treatment to produce the aggregate filler (d-1).

The surface treatment is as described above.

The specific surface area of aggregate filler (d-1) can be measured using a BET (Brunauer Emmett Teller) multiple-point method.

The method of measurement of specific surface area is as described in the EXAMPLES section below. Preferably, the specific surface area of aggregate filler (d-1) is measured in an untreated state, without surface treatment.

Regarding the analysis of the measurement results, it can be conducted using a multi-point BET method with 5 points on the adsorption isotherm where the ratio (P/P0) of adsorption equilibrium pressure P (kPa) to saturation vapor pressure P0 (kPa) is 0.05 to 0.3.

The inorganic filler used as aggregate filler (d-1) may be a commercially available product.

Examples of such commercially available products include SG-SZ200G151CMP8, SG-SZ50G151CMP8, and SG-SZ200G154CMP8 (manufactured by Sukgyung AT).

In view of properties such as the ease of polishing, adhesive properties to tooth structure, and mechanical strength of the dental composition obtained, the content of aggregate filler (d-1) is 20 to 85 mass%, preferably 30 to 80 mass%, more preferably 35 to 75 mass%, even more preferably 40 to 75 mass%, particularly preferably 45 to 75 mass% in total 100 mass% of the dental composition.

### · Filler (d-2) Other Than Aggregate Filler (d-1)

In view of adjusting handling properties and providing high mechanical strength, the dental composition preferably comprises a filler (d-2) other than aggregate filler (d-1) (hereinafter, also referred to simply as "filler (d-2)").

Examples of filler (d-2) include inorganic fillers, organic-inorganic composite fillers, and organic fillers. The filler (d-2) may be used alone, or two or more thereof may be used in combination.

In view of properties such as the handling properties and mechanical strength of the dental composition obtained, the inorganic filler as the filler (d-2) other than aggregate filler (d-1) has an average particle diameter of preferably 0.001 to 50 µm, more preferably 0.001 to 20 µm, even more preferably 0.005 to 10 µm.

In the present invention, the average particle diameter of filler (d-2) means the average particle diameter before surface treatment when filler (d-2) is subjected to surface treatment as above. A certain preferred embodiment is, for example, a dental composition in which the filler (d-2) is an inorganic filler.

In view of properties such as the ease of polishing and mechanical strength of the dental composition obtained, the content of the filler (d-2) other than aggregate filler (d-1) is preferably 0 to 50 mass%, more preferably 1 to 45 mass%, even more preferably 2 to 40 mass% in 100 mass% of the dental composition.

In certain embodiments, the filler (d-2) other than aggregate filler (d-1) may be absent.

The content of filler (d) is not particularly limited. However, in view of handling properties, ease of polishing, the mechanical strength of the cured product, and adhesive properties to tooth structure, the content of filler (d) is preferably 50 to 90 mass%, more preferably 55 to 85 mass%, even more preferably 60 to 80 mass%, particularly preferably 60 to 78 mass% in 100 mass% of the dental composition.

### <Polymerization Accelerator (e)>

In view of the mechanical strength of the cured product, the dental composition preferably comprises a polymerization accelerator (e), along with the water-soluble photopolymerization initiator (c-1a), water-insoluble photopolymerization initiator (c-1b), and chemical polymerization initiator (c-2).

Examples of the polymerization accelerator (e) that can be used in the present invention include amines, sulfinic acid and salts thereof, borate compounds, derivatives of barbituric acid, triazine compounds, copper compounds, tin compounds, vanadium compounds, halogen compounds, aldehydes, thiol compounds, sulfites, bisulfites, and thiourea compounds. The polymerization accelerator (e) may be used alone, or two or more thereof may be used in combination.

The amines used as polymerization accelerator (e) can be categorized into aliphatic amines and aromatic amines. Examples of the aliphatic amines include primary aliphatic amines such as n-butylamine, n-hexylamine, and n-octylamine; secondary aliphatic amines such as diisopropylamine, dibutylamine, and N-methylethanolamine; and tertiary aliphatic amines such as N-methyldiethanolamine, N-ethyldiethanolamine, N-n-butyldiethanolamine, N-lauryldiethanolamine, 2-(dimethylamino)ethyl methacrylate, N-methyldiethanolamine dimethacrylate, N-ethyldiethanolamine dimethacrylate, triethanolamine monomethacrylate, triethanolamine dimethacrylate, triethanolamine trimethacrylate, triethanolamine, trimethylamine, triethylamine, and tributylamine. In view of the curability and storage stability of the dental composition, preferred are tertiary aliphatic amines, more preferably N-methyldiethanolamine and triethanolamine.

Examples of the aromatic amines include N,N-bis(2-hydroxyethyl)-3,5-dimethylaniline, N,N-bis(2-hydroxyethyl)-p-toluidine, N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-t-butylaniline, N,N-bis(2-hydroxyethyl)-3,5-diisopropylaniline, N,N-bis(2-hydroxyethyl)-3,5-di-t-butylaniline, N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, N,N-dimethyl-3,5-di-t-butylaniline, ethyl 4-(N,N-dimethylamino)benzoate, methyl 4-(N,N-dimethylamino)benzoate, propyl 4-(N,N-dimethylamino)benzoate, n-butoxyethyl 4-(N,N-dimethylamino)benzoate, 2-(methacryloyloxy)ethyl 4-(N,N-dimethylamino)benzoate, 4-(N,N-dimethylamino)benzophenone, and butyl 4-(N,N-dimethylamino)benzoate. In view of the ability to impart excellent curability to the dental composition, it is preferable to use at least one selected from the group consisting of N,N-bis(2-hydroxyethyl)-p-toluidine, ethyl 4-(N,N-dimethylamino)benzoate, n-butoxyethyl 4-(N,N-dimethylamino)benzoate, and 4-(N,N-dimethylamino)benzophenone.

Specific examples of sulfinic acid and salts thereof, borate compounds, barbituric acid derivatives, triazine compounds, copper compounds, tin compounds, vanadium compounds, halogen compounds, aldehydes, thiol compounds, sulfites, bisulfites, and thiourea compounds include those mentioned in WO2008/087977.

The content of the polymerization accelerator (e) used in the present invention is not particularly limited. However, in view of the curability and other properties of the dental composition, the content of polymerization accelerator (e) is preferably 0.001 to 30 parts by mass, more preferably 0.01 to 20 parts by mass, even more preferably 0.1 to 5 parts by mass relative to total 100 parts by mass of monomers in the dental composition. When the content of polymerization accelerator (e) is at or above these lower limits, it is easier to provide a sufficient adhesive strength by allowing polymerization to sufficiently proceed. When the content of polymerization accelerator (e) is at or below the foregoing upper limits, it is easier to provide sufficient adhesive properties, and to reduce precipitation of the polymerization accelerator (e) itself from the dental composition.

### <Fluorine-Ion Releasing Substance>

The dental composition may additionally comprise a fluorine-ion releasing substance. By containing a fluorine-ion releasing substance, the dental composition produced can impart acid resistance to tooth structure.

Examples of the fluorine-ion releasing substance include fluorine-ion releasing polymers such as copolymers of methyl methacrylate and methacrylic acid fluoride; and metal fluorides such as sodium fluoride, potassium fluoride, sodium monofluorophosphate, lithium fluoride, and ytterbium fluoride.

The fluorine-ion releasing substance may be used alone, or two or more thereof may be used in combination.

The dental composition may also comprise a known additive, provided that such additives do not cause a performance drop. Examples of such additives include polymerization inhibitors, antioxidants, colorants (pigments, dyes), ultraviolet absorbers, fluorescent agents, solvents such as organic solvents, and thickeners. The additives may be used alone, or two or more thereof may be used in combination.

In certain embodiments, the content of the solvent (for example, water, organic solvent) in the dental composition is preferably less than 1 mass%, more preferably less than 0.1 mass%, even more preferably less than 0.01 mass% in total 100 mass% of the dental composition.

In view of adjusting storage stability and curability, a dental composition of the present invention preferably comprises a polymerization inhibitor. Examples of the polymerization inhibitor include hydroquinone, hydroquinone monomethyl ether, dibutylhydroquinone, dibutylhydroquinone monomethyl ether, t-butylcatechol, 2-t-butyl-4,6-dimethylphenol, 2,6-di-t-butylphenol, and 3,5-di-t-butyl-4-hydroxytoluene. These may be used alone, or two or more thereof may be used in combination.

The content of the polymerization inhibitor is preferably 0.001 to 1.0 parts by mass relative to total 100 parts by mass of monomers in the dental composition.

In view of photostability against ambient light from light sources such as fluorescent lamps and LEDs, and reducing discoloration of the cured product, a dental composition of the present invention preferably comprises an ultraviolet absorber.

Examples of the ultraviolet absorber include benzotriazole compounds such as 2-(2-hydroxyphenyl)benzotriazole, 2-(2-hydroxy-5-methylphenyl)benzotriazole, 2-(2-hydroxy-5-ethylphenyl)benzotriazole, 2-(2-hydroxy-5-propylphenyl)benzotriazole, 2-(2-hydroxy-5-tert-octylphenyl)benzotriazole, and 2-(3-tert-butyl-2-hydroxy-5-methylphenyl)-5-chloro-2H-benzotriazole (Tinuvin 326); and benzoimidazole compounds. Preferred is Tinuvin 326. These may be used alone, or two or more thereof may be used in combination.

From an aesthetic perspective to reproduce a shade close to that of tooth structure, a dental composition of the present invention preferably comprises a fluorescent agent. While known fluorescent agents can be used without any limitations, phthalic acid ester fluorescent agents are preferred.

Specific examples of phthalic acid ester fluorescent agents include dimethyl 2,5-dihydroxyterephthalate, diethyl 2,5-dihydroxyterephthalate, dimethylaminoterephthalate, and diethylaminoterephthalate. More preferred are phthalic acid ester fluorescent agents substituted with hydroxyl groups, such as diethyl 2,5-dihydroxyterephthalate.

The fluorescent agent may be used alone, or two or more thereof may be used in combination.

The content of the fluorescent agent is not particularly limited. However, a certain quantity of fluorescent agent is necessary to ensure fluorescence. If the quantity is too high, it tends to cause difficulty in ensuring photostability.

For a good balance between photostability and fluorescence, the content of the fluorescent agent is preferably 0.005 to 0.5 parts by mass, more preferably 0.01 to 0.1 parts by mass relative to total 100 parts by mass of monomers in the dental composition.

In order to reproduce a shade close to that of tooth structure, a dental composition of the present invention preferably comprises a colorant. The type of colorant is not particularly limited, and inorganic pigments and/or organic pigments can be used without restrictions, according the shade desired for the dental composition and its cured product. The colorant may be used alone, or two or more thereof may be used in combination.

The colorant is a component added to the dental composition in trace amounts. For each type of colorant, the colorant content is less than 1.0 mass% in total 100 mass% of the dental composition.

The colorant may be one with a refractive index of more than 2.00. The colorant may have a refractive index of 2.05 or more, or 2.10 or more.

The shape of colorant is not particularly limited, and the colorant may have any particle shape, including spherical, needle-shape, plate-shape, crushed shape, and scale-like, without any restrictions.

Examples of the inorganic pigments include:
chromates such as chrome yellow, zinc yellow, and barium yellow;
ferrocyanides such as iron blue;
sulfides such as silver vermilion, cadmium yellow, zinc sulfide, and cadmium red;
sulfates such as barium sulfate, zinc sulfate, and strontium sulfate;
oxides such as zinc white, antimony white, titanium white, red iron oxide, iron black, and chromium oxide;
hydroxides such as aluminum hydroxide;
silicates such as calcium silicate, and ultramarine; and
carbons such as carbon black, and graphite.

Examples of the organic pigments include:
nitroso pigments such as naphthol green B, and naphthol green Y;
nitro pigments such as naphthol yellow S, and xylene fast yellow 2G;
insoluble azo pigments such as toluidine red 4R, brilliant fast scarlet, Hansa yellow, and pigment yellow;
poorly soluble azo pigments such as lithol red, lake red C, and lake red D;
soluble azo pigments such as brilliant carmine 6B, toluidine red F5R, pigment scarlet 3B, and Bordeaux 10B;
phthalocyanine pigments such as phthalocyanine blue, phthalocyanine green, and sky blue;
basic dye pigments such as rhodamine lake, malachite green lake, and methyl violet lake; and
acidic dye pigments such as peacock blue lake, eosin lake, quinoline yellow lake, and aluminum lake.

Among these colorants, inorganic pigments such as titanium white, red iron oxide, iron black, and yellow ferrous oxide are preferred over the organic pigments due to their superior heat resistance and lightfastness.

The content of the colorant in a dental composition of the present invention is not particularly limited, as long as it falls within the effective range of the present invention. However, in view of aesthetics, the colorant content is preferably 0.0005 parts or more by mass, more preferably 0.002 parts or more by mass, even more preferably 0.004 parts or more by mass, particularly preferably 0.006 parts or more by mass relative to total 100 parts by mass of monomers in the dental composition. When the colorant content is at or above these lower limits, the filled area created by filling the dental composition into cavities can be prevented from appearing dark and dull.

The colorant content is preferably 2.0 parts or less by mass, more preferably 1.0 part or less by mass, even more preferably 0.5 parts or less by mass, particularly preferably 0.3 parts or less relative to total 100 parts by mass of monomers in the dental composition. When the colorant content is at or below these upper limits, the filled area can effectively reflect the shade of natural teeth at the floor of cavities.

The colorant content is preferably 0.00001 mass% or more, more preferably 0.0001 mass% or more, even more preferably 0.0005 mass% or more, particularly preferably 0.001 mass% or more in total 100 mass% of the dental composition. The colorant content is preferably 0.5 mass% or less, more preferably 0.3 mass% or less, even more preferably 0.1 mass% or less, particularly preferably 0.07 mass% or less.

A dental composition of the present invention has a viscosity (shear viscosity) ranging preferably from 1 to 200 Pa·s, more preferably from 5 to 150 Pa·s, even more preferably from 10 to 120 Pa·s as measured at 25°C with a shear rate of 10 s⁻¹.

The method of viscosity measurement is as described in the EXAMPLES section below.

A dental composition of the present invention can be suitably used in dental treatment applications such as self-adhesive dental composite resins, and dental cements.

Examples of preferred composition ratios for self-adhesive dental composite resins are as follows.

When the total amount of monomers is 100 parts by mass, it is preferable to comprise 1 to 40 parts by mass of monomer (a) having an acidic group, and 60 to 99 parts by mass of monomer (b) having no acidic group in total 100 parts by mass of the monomers, along with 0.05 to 10 parts by mass of photopolymerization initiator (c-1), 20 to 500 parts by mass of aggregate filler (d-1), 80 to 400 parts by mass of filler (d-2), and 0.001 to 30 parts by mass of polymerization accelerator (e) relative to total 100 parts by mass of the monomers, more preferably 2.5 to 35 parts by mass of monomer (a) having an acidic group, and 65 to 97.5 parts by mass of monomer (b) having no acidic group in total 100 parts by mass of the monomers, along with 0.1 to 5 parts by mass of photopolymerization initiator (c-1), 30 to 400 parts by mass of aggregate filler (d-1), 90 to 300 parts by mass of filler (d-2), and 0.01 to 10 parts by mass of polymerization accelerator (e) relative to total 100 parts by mass of the monomers, even more preferably 5 to 30 parts by mass of monomer (a) having an acidic group, and 70 to 95 parts by mass of monomer (b) having no acidic group in total 100 parts by mass of the monomers, along with 0.15 to 2.5 parts by mass of photopolymerization initiator (c-1), 40 to 300 parts by mass of aggregate filler (d-1), 100 to 200 parts by mass of filler (d-2), and 0.1 to 5 parts by mass of polymerization accelerator (e) relative to total 100 parts by mass of the monomers.

Examples of preferred composition ratios for dental cements are as follows.

When the total amount of monomers is 100 parts by mass, it is preferable to comprise 1 to 40 parts by mass of monomer (a) having an acidic group, and 60 to 99 parts by mass of monomer (b) having no acidic group in total 100 parts by mass of the monomers, along with 0.05 to 5 parts by mass of photopolymerization initiator (c-1), 0.01 to 20 parts by mass of chemical polymerization initiator (c-2), 20 to 500 parts by mass of aggregate filler (d-1), 80 to 400 parts by mass of filler (d-2), and 0.001 to 30 parts by mass of polymerization accelerator (e) relative to total 100 parts by mass of the monomers, more preferably 2.5 to 35 parts by mass of monomer (a) having an acidic group, and 65 to 97.5 parts by mass of monomer (b) having no acidic group in total 100 parts by mass of the monomers, along with 0.1 to 3 parts by mass of photopolymerization initiator (c-1), 0.1 to 10 parts by mass of chemical polymerization initiator (c-2), 30 to 400 parts by mass of aggregate filler (d-1), 90 to 300 parts by mass of filler (d-2), and 0.01 to 10 parts by mass of polymerization accelerator (e) relative to total 100 parts by mass of the monomers, even more preferably 5 to 30 parts by mass of monomer (a) having an acidic group, and 70 to 95 parts by mass of monomer (b) having no acidic group in total 100 parts by mass of the monomers, along with 0.15 to 2 parts by mass of photopolymerization initiator (c-1), 0.15 to 5 parts by mass of chemical polymerization initiator (c-2), 40 to 300 parts by mass of aggregate filler (d-1), 100 to 200 parts by mass of filler (d-2), and 0.1 to 5 parts by mass of polymerization accelerator (e) relative to total 100 parts by mass of the monomers.

A dental composition comprising a monomer (a) having an acidic group, a monomer (b) having no acidic group, a polymerization initiator (c), and a filler (d) (aggregate filler (d-1)) along with optional components can be produced with ease using methods known to a person skilled in the art.

A certain embodiment is, for example, a method for producing a dental composition by mixing at least three of the monomer (a) having an acidic group, the monomer (b) having no acidic group, the polymerization initiator (c), and the aggregate filler (d-1).

When producing a dental composition optionally comprising additional components (such as the filler (d-2) other than aggregate filler (d-1), and the polymerization accelerator (e)), the method of the above embodiment may additionally comprise mixing the filler (d-2) other than aggregate filler (d-1), and the polymerization accelerator (e).

A dental composition of the present invention may be combined with materials such as dental etchants, dental primers, and dental bonding materials.

In view of adhesive strength to tooth structure, it is preferable to combine dental primers and dental bonding materials, more preferably dental bonding materials.

The dental primers and dental bonding materials may or may not be polymerized alone. However, in view of adhesive properties to tooth structure, it is more preferable to polymerize these by photopolymerization or chemical polymerization. The dental primers and dental bonding materials may be used individually or in combination. In view of adhesive properties to tooth structure, it is preferable to employ a procedure that applies dental bonding materials after the application of dental primers.

When the dental composition is used with materials such as dental etchants, dental primers, and dental bonding materials, these are used before applying the dental composition.

### EXAMPLES

The following describes the present invention in detail using Examples and Comparative Examples. However, the present invention is not limited to the following Examples. In the following, "part(s)" means "part(s) by mass", unless otherwise specifically stated.

The components of the dental compositions of Examples and Comparative Examples are presented below, along with the abbreviations.
[Monomer (a) having an acidic group]
MDP: 10-methacryloyloxydecyl dihydrogen phosphate
GPDM: 1,3-dimethacryloyloxypropyl-2-dihydrogen phosphate
[Monomer (b) having no acidic group]
D-2.6E: 2,2-bis(4-methacryloyloxypolyethoxyphenyl)propane (with an average of 2.6 moles of ethoxy group added)
POB-MA: phenoxybenzyl methacrylate
1-NMMA: 1-naphthylmethyl methacrylate
MAEA: N-methacryloyloxyethylacrylamide
THF-MA: tetrahydrofurfuryl methacrylate
UDMA: 2,2,4-trimethylhexamethylenebis(2-carbamoyloxyethyl)dimethacrylate

UN7600: urethane acrylate (manufactured by Negami Chemical Industrial Co., Ltd.; weight-average molecular weight (Mw): 11,500, glass transition temperature (Tg): - 42°C and 44.6°C, number of polymerizable groups: 2, weight-average molecular weight per polymerizable group: 5,750)

Note that the weight-average molecular weight (Mw) refers to a weight-average molecular weight measured by gel permeation chromatography (GPC) in terms of polystyrene.

### [Photopolymerization Initiator (c-1)]

- Water-soluble photopolymerization initiator (c-1a)
   BAPO-ONa: sodium bis(2,4,6-trimethylbenzoyl)phosphinate
- Water-insoluble photopolymerization initiator (c-1b)
   CQ: camphorquinone

### [Filler (d)]

### · Aggregate filler (d-1)

### Filler 1: Surface-treated SiO₂-ZrO₂ aggregate filler

Surface-treated SiO₂-ZrO₂ aggregate filler (SG-SZ200G151CMP8, average particle diameter of primary particles: 200 nm, average particle diameter of secondary particles: 5.2 µm, refractive index: 1.51; manufactured by Sukgyung AT)

### Filler 2: Surface-treated SiO₂-ZrO₂ aggregate filler

Surface-treated SiO₂-ZrO₂ aggregate filler (SG-SZ50G151CMP8, average particle diameter of primary particles: 50 nm, average particle diameter of secondary particles: 6.5 µm, refractive index: 1.51; manufactured by Sukgyung AT)

### Filler 3: Surface-treated BaO aggregate filler

A three-neck flask was charged with 100 parts by mass of barium glass (trade name: GM27884, average particle diameter of primary particles: 180 nm; manufactured by Schott), 22 parts by mass of 3-methacryloyloxypropyltrimethoxysilane, and 500 mL of a 0.5% acetic acid aqueous solution. These were then stirred for 2 hours at room temperature under ultrasonic dispersion to prepare a slurry solution.

Subsequently, the slurry solution was granulated by spraying it with a spray dryer (FOC-16, Ohkawara Kakohki Co., Ltd.). The granulated filler was then heated at 700°C for 1 hour using an electric furnace to prepare a BaO aggregate filler having an average particle diameter of 7.8 µm in terms of secondary particles.

Thereafter, 100 parts by mass of the BaO aggregate filler was charged into a three-neck flask along with 11 parts by mass of 3-methacryloyloxypropyltrimethoxysilane and 500 mL of a 0.5% acetic acid aqueous solution. These were then stirred for 2 hours at room temperature under ultrasonic dispersion. After freeze drying to remove water, the mixture was heat-treated at 90°C for 3 hours to obtain filler 3.

### · Filler (d-2) other than aggregate filler (d-1)

### Filler 4: Surface-treated SiO₂-treated silica

A three-neck flask was charged with 100 g of Ar130 (hydrophilic fumed silica, ultrafine particulate silica Aerosil^{®} 130, average particle diameter: 16 nm, refractive index: 1.46; manufactured by Nippon Aerosil Co., Ltd.), 30 g of 3-methacryloyloxypropyltrimethoxysilane, and 200 mL of a 0.3 mass% acetic acid aqueous solution. These were then stirred for 2 hours at room temperature under ultrasonic dispersion. After freeze drying to remove water, the mixture was heat-treated at 90°C for 3 hours to obtain filler 4.

### Filler 5: Surface-treated SiO₂-coated YbF₃

Surface-treated SiO₂-coated YbF₃ (SG-YBF100WSCMP10, average particle diameter of primary particles: 110 nm, average particle diameter of secondary particles: 1.2 µm, refractive index: 1.53; manufactured by Sukgyung AT)

### Filler 6: Surface-treated SiO₂ filler

A silica stone powder (trade name Hi-Silica, refractive index: 1.55; manufactured by Nitchitsu Co., Ltd.) was pulverized with a dry ball mill (10-mm diameter alumina balls) to obtain a pulverized silica stone powder. The pulverized silica stone powder had an average particle diameter of 2.2 µm as measured by volume with a laser diffraction particle size distribution analyzer (Model SALD-2300, manufactured by Shimadzu Corporation). Subsequently, 100 g of this pulverized silica stone powder was charged into a three-neck flask along with 4 g of 3-methacryloyloxypropyltrimethoxysilane, and 200 mL of an acetic acid aqueous solution. These were stirred for 2 hours at room temperature under ultrasonic dispersion. After freeze drying to remove water, the mixture was heat-treated at 90°C for 3 hours to obtain filler 6.

### Filler 7: Surface-treated aggregate SiO₂ filler

A three-neck flask was charged with 100 parts by mass of Silica Micro Bead P-500 (average particle diameter of primary particles: 12 nm, average particle diameter of secondary particles: 2.0 µm, refractive index: 1.46; manufactured by JGC C & C), 20 parts by mass of 3-methacryloyloxypropyltrimethoxysilane, and 200 mL of a 0.5% acetic acid aqueous solution. These were stirred for 2 hours at room temperature under ultrasonic dispersion. After freeze drying to remove water, the mixture was heat-treated at 90°C for 3 hours to obtain filler 7.

### Filler 8: Surface-treated BaO filler

A three-neck flask was charged with 100 parts by mass of barium glass (trade name: GM27884, average particle diameter of primary particles: 0.18 µm; manufactured by Schott), 11 parts by mass of 3-methacryloyloxypropyltrimethoxysilane, and 500 mL of a 0.5% acetic acid aqueous solution. These were stirred for 2 hours at room temperature under ultrasonic dispersion. After freeze drying to remove water, the mixture was heat-treated at 90°C for 3 hours to obtain filler 8.

Table 1 outlines the properties of the fillers 1 to 8. The elemental compositions shown in Table 1 are based on the metal elements (excluding fluorine) found in the product brochures provided by the manufacturers of the filler raw materials or the fillers themselves. In addition, the average particle diameters of primary particles and secondary particles refer to either the values found in the product brochures provided by the manufacturers of the filler raw materials or the fillers themselves, or volumetrically measured values obtained using a laser diffraction particle size distribution analyzer (Model SALD-2300 manufactured by Shimadzu Corporation) with a 0.2% sodium hexametaphosphate aqueous solution used as dispersion medium. The specific surface area refers to values measured by the multi-point BET method described below, or values taken from the product brochures or test reports provided by the manufacturers of the fillers themselves.

### [Measurement Method of Specific Surface Area]

The specific surface area was measured using a multi-point BET method.

Specifically, the aggregate filler was degassed in a vacuum at 100°C for 2 hours, and the specific surface area was measured according to the BET method with a specific surface area measurement device (BELSORP-mini II manufactured by MicrotracBEL Corp.), using nitrogen as adsorbate gas and at a measurement temperature of 77 K.

The measurement results were analyzed following the multi-point BET method by taking 5 points on the adsorption isotherm where the ratio (P/P0) of adsorption equilibrium pressure P (kPa) to saturation vapor pressure P0 (kPa) is 0.05 to 0.3.

The results are presented in Table 1.

**[Table 1]**

| Filler | Average particle diameter of primary particles (nm) | Average particle diameter of secondary particles (µm) | Specific surface area (m²/g) | Elemental composition |
|---|---|---|---|---|
| Filler 1 | 200 | 5.2 | 26 | Si, Zr |
| Filler 2 | 20 | 6.5 | 53 | Si, Zr |
| Filler 3 | 180 | 7.8 | 16 | Ba, Al, B, Si |
| Filler 4 | 16 | - | 130 | Si |
| Filler 5 | 120 | 1.2 | 4 | Si, Yb, F |
| Filler 6 | 2,200 | - | 0.5 | Si |
| Filler 7 | 12 | 2 | 99 | Si |
| Filler 8 | 180 | - | 40 | Ba, Al, B, Si |

### [Polymerization Accelerator (e)]

DABE: ethyl 4-(N,N-dimethylamino)benzoate

### [Others]

TN326: 2-(3-tert-butyl-2-hydroxy-5-methylphenyl)-5-chloro-2H-benzotriazole (trade name: Tinuvin 326, manufactured by BASF Japan) (ultraviolet absorber)

LBL: diethyl 2,5-dihydroxyterephthalate (fluorescent agent)

BHT: 3,5-di-t-butyl-4-hydroxytoluene (polymerization inhibitor)

### [Examples 1 to 13 and Comparative Examples 1 to 4]

### [Preparation of Dental Composition]

The raw materials shown in Table 2 were mixed and kneaded at ordinary temperature (23°C) in the absence of light, and the resulting uniform mixture was subjected to vacuum defoaming to yield dental compositions in paste form.

### Test Example 1: Ease of Polishing

Following vacuum defoaming, the dental composition was placed in a Teflon^{®} mold (10 mm in diameter, 2.0 mm in thickness). With the upper and lower sides compressed with glass slides, the dental composition was cured into a sample by exposing only the upper side to light for 10 seconds, using a dental polymerization LED photoirradiator (trade name PenCure 2000, manufactured by J. Morita Corp.) (n = 3). The sample was then removed from the Teflon^{®} mold, and the clean smooth upper surface was ground with #600 abrasive paper under dry conditions. Subsequently, the smooth surface was polished at a rotational speed of approximately 5,000 rpm for 15 seconds, using the laboratory engine Volvere RX (manufactured by Nakanishi Inc.) with a CompoMaster CA (manufactured by Shofu Inc.) under water irrigation. The gloss of the polished surface was then measured as a percentage relative to a mirror serving as 100%, using a gloss meter (VG 7000 manufactured by Nippon Denshoku Industries Co., Ltd.). The measurement angle was set at 60°.

The preferred glossiness is 50% or more, more preferably 55% or more, even more preferably 60% or more, particularly preferably 65% or more.

### Test Example 2: Shear Adhesive Strength (Enamel, Dentin)

The test was conducted in compliance with ISO29022:2013, specifically as follows.

The labial surfaces of bovine teeth were polished with #80 silicon carbide paper (manufactured by Nihon Kenshi Co., Ltd.) under running water to obtain samples with exposed flat enamel surfaces and samples with exposed flat dentin surfaces.

First, samples with exposed flat enamel surfaces were used to measure shear adhesive strength to enamel, as follows.

A mold with 15 holes (15-hole mold, manufactured by Ultradent Products Inc., 35 mm in diameter × 25 mm in height) was separately prepared and a tape was attached to the bottom of the mold. The bovine tooth sample was then secured onto the tape.

Thereafter, a resin for dental impression trays (Tray Resin II manufactured by Shofu Inc. under this trade name) was filled into the mold, and left to stand for about 30 minutes to cure. This resulted in a composite of bovine tooth and cured resin. This composite was then removed from the mold as a sample.

The composite had the bovine tooth exposed at the top surface of the cured resin. The top surface of the sample was polished under running water to a size large enough to provide an adhesive surface (a diameter of 2.38 mm or more), using a #600 silicon carbide paper (manufactured by Nihon Kenshi Co., Ltd.), and the adhesive surface was ultrasonically washed with water for 5 minutes.

Subsequently, a separately prepared CR filling mold (Bonding Mold Insert, manufactured by Ultradent Products Inc.; Ø = 2.38 mm) was installed on a dedicated instrument (Bonding Clamp, manufactured by Ultradent Products Inc.). The sample was then secured by lowering the CR filling mold, allowing the CR filling mold installed on the dedicated instrument to contact the adhesive surface of the sample.

Thereafter, the prepared dental composition was filled into the hole of the CR filling mold to form a thin layer of no greater than 1 mm. After filling another portion into the CR filling mold (to about 2/3 of the mold, or about 2 mm thick), the dental composition was left to stand for 10 seconds, and irradiated with light for 10 seconds to cure, using a dental polymerization photoirradiator (manufactured by Ultradent Products Inc. under the trade name VALO). The sample was removed from the CR filling mold, and used as an adhesion test sample. A total of ten adhesion test samples were prepared.

The adhesion test sample was immersed in distilled water inside a container, and was left to stand for 24 hours in this state inside a thermostatic chamber with the chamber temperature set to 37°C. The sample was immediately measured for its shear adhesive strength to enamel after being taken out of distilled water.

For the measurement of adhesive strength (shear adhesive strength), the adhesion test sample was installed on a dedicated holder (Test Base Clamp, manufactured by Ultradent Products Inc.), and the adhesive strength was measured using a dedicated jig (Crosshead Assembly, manufactured by Ultradent Products Inc.) and a universal testing machine (manufactured by Shimadzu Corporation) with the crosshead speed set at 1 mm/min. The average values are presented in the Table (n = 10).

The shear adhesive strength to dentin was also measured in the same manner. The average values are presented in the Table (n = 10).

In view of adhesive strength to tooth structure, larger values are preferred for shear adhesive strength to both enamel and dentin. The shear adhesive strength to enamel and dentin is preferably more than 4.0 MPa, more preferably 5.0 Mpa or more, even more preferably 6.0 Mpa or more, particularly preferably 8.0 Mpa or more.

### Test Example 3: Flexural Properties (Flexural Strength)

The flexural modulus and flexural strength were evaluated by conducting a flexure test in compliance with ISO 4049:2019, specifically as follows.

The prepared paste-form dental composition was filled into a SUS mold (2 mm in length × 25 mm in width × 2 mm in thickness), and pressed with glass slides from the top and bottom (over a 2 mm × 25 mm surface). Under the pressure of the glass slides, the paste was exposed to light through the glass slides, using a dental polymerization LED photoirradiator (PenCure 2000, manufactured by J. Morita Corp.). Here, light was applied for 10 seconds from both sides of the paste in standard mode, at five different points on each side (a total of 50 seconds of light exposure per side). This process cured the paste and produced a cured product. A total of five cured products were prepared for each Example and Comparative Example.

The samples were immersed in distilled water in a container, and left to stand in this state for 24 hours inside a thermostatic chamber with the chamber temperature set to 37°C. The three-point flexural strength was then measured at a span length of 20 mm and a crosshead speed of 1 mm/min, using a universal testing machine (Autograph AG-I 100kN, manufactured by Shimadzu Corporation) (n = 5), and the results were averaged.

In view of the mechanical strength of the cured product, the preferred flexural strength is 90 Mpa or more, more preferably 95 Mpa or more, even more preferably 100 Mpa or more, particularly preferably 105 Mpa or more, most preferably 110 Mpa or more.

### Test Example 4: Shear Viscosity

The paste-form dental composition according to each Example and Comparative Example was measured for shear viscosity using a dynamic viscoelasticity meter (rotational rheometer AR2000 manufactured by TA instrument). The measurements were carried out with a 25 mm plate diameter, a plate gap of 0.50 mm, and a shear rate of 10 s⁻¹ at 25°C (n = 3), and the results were averaged.

The preferred viscosity when it is measured at 25°C with a shear rate of 10 s⁻¹ ranges from 1 to 200 Pa·s, more preferably 5 to 150 Pa·s, even more preferably 10 to 120 Pa·s.

**[Table 2]**

| Components (parts by mass) | | Ex. 1 | Ex. 2 | Ex. 3 | Ex. 4 | Ex. 5 | Ex. 6 | Ex. 7 | Ex. 8 | Ex. 9 | Ex. 10 | Ex. 11 | Ex. 12 | Ex. 13 | Com. Ex. 1 | Com. Ex. 2 | Com. Ex. 3 | Com. Ex. 4 |
|---|---|---|---|---|---|---|---|---|---|---|---|---|---|---|---|---|---|---|
| Monomer (a) having acidic group | MDP | 5 | 5 | 5 | 5 | 5 | 5 | | 25 | 5 | 5 | 5 | 5 | 5 | 5 | 5 | 5 | 0 |
| | GPDM | | | | | | | 5 | | | | | | | | | | |
| Monomer (b) having no acidic group | D-2.6E | 27.5 | 57.5 | 27.5 | 27.5 | 27.5 | 27.5 | 27.5 | 20 | 27.5 | | | | | 57.5 | 27.5 | 27.5 | 27.5 |
| | POB-MA | | | | | | | | | | 27.5 | | | | | | | |
| | 1-NMMA | | | | | | | | | | | 27.5 | 27.5 | 27.5 | | | | |
| | UDMA | 30 | | 30 | 30 | 30 | 30 | 30 | 20 | 30 | 30 | 30 | 30 | 30 | | 30 | 30 | 35 |
| | MAEA | 5 | 5 | 5 | 5 | 5 | 5 | 5 | 5 | 5 | 5 | 5 | 22.5 | 22.5 | 5 | 5 | 5 | 5 |
| | THF-MA | 15 | 15 | 15 | 15 | 15 | 15 | 15 | 15 | 15 | 15 | 15 | 15 | 15 | 15 | 15 | 15 | 15 |
| | UN7600 | 17.5 | 17.5 | 17.5 | 17.5 | 17.5 | 17.5 | 17.5 | 17 | 17.5 | 17.5 | 17.5 | | | 17.5 | 17.5 | 17.5 | 17.5 |
| Polymerization initiator (c) | CQ | 0.2 | 0.2 | 0.2 | 0.2 | 0.2 | 0.2 | 0.2 | 0.2 | 0.2 | 0.2 | 0.2 | 0.2 | 0.2 | 0.2 | 0.2 | 0.2 | 0.2 |
| | BAPo-ONa | 0.3 | 0.3 | | 0.3 | 0.3 | 0.3 | 0.3 | 0.3 | 0.3 | 0.3 | 0.3 | 0.3 | 0.7 | 0.3 | 0.3 | 0.3 | 0.3 |
| Aggregate filler (d-1) | Filler 1 | 160 | 160 | 160 | 200 | | | 160 | 160 | 270 | 160 | 160 | 160 | 160 | | | | 160 |
| | Filler 2 | | | | | 120 | | | | | | | | | | | | |
| | Filler 3 | | | | | | 120 | | | | | | | | | | | |
| Filler (d-2) other than aggregate filler (d-1) | Filler 4 | 5 | 5 | 5 | | 5 | 5 | 5 | 5 | | 5 | 5 | 5 | 5 | 5 | 5 | 5 | 5 |
| | Filler 5 | 40 | 40 | 40 | | 40 | 40 | 40 | 40 | | 40 | 40 | 40 | 40 | 40 | 40 | 40 | 40 |
| | Filler 6 | | | | | | | | | | | | | | 190 | | | |
| | Filler 7 | | | | | | | | | | | | | | | 100 | | |
| | Filler 8 | | | | | | | | | | | | | | | | 110 | |
| Polymerization accelerator (e) | DABE | 0.2 | 0.2 | 0.3 | 0.2 | 0.2 | 0.2 | 0.2 | 0.2 | 0.2 | 0.2 | 0.2 | 0.2 | 0.2 | 0.2 | 0.2 | 0.2 | 0.2 |
| Others | TN326 | 0.2 | 0.2 | 0.2 | 0.2 | 0.2 | 0.2 | 0.2 | 0.2 | 0.2 | 0.2 | 0.2 | 0.2 | 0.2 | 0.2 | 0.2 | 0.2 | 0.2 |
| | LBL | 0.02 | 0.02 | 0.02 | 0.02 | 0.02 | 0.02 | 0.02 | 0.02 | 0.02 | 0.02 | 0.02 | 0.02 | 0.02 | 0.02 | 0.02 | 0.02 | 0.02 |
| | BHT | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 |
| Ease of polishing | % | 70 | 68 | 65 | 72 | 60 | 55 | 70 | 66 | 77 | 68 | 71 | 72 | 76 | 4 | 55 | 68 | 70 |
| Shear adhesive strength (enamel) | MPa | 12 | 10 | 10 | 10 | 9 | 11 | 10 | 12 | 7 | 8 | 7 | 9 | 10 | 7 | 8 | 8 | 1 |
| Shear adhesive strength (dentin) | MPa | 8 | 7 | 6 | 8 | 6 | 8 | 6 | 7 | 6 | 7 | 6 | 10 | 14 | 9 | 3 | 4 | 1 |
| Flexural strength | MPa | 121 | 125 | 113 | 129 | 109 | 124 | 118 | 100 | 134 | 103 | 110 | 120 | 135 | 100 | 123 | 82 | 126 |
| Shear viscosity | s⁻¹ | 50 | 62 | 50 | 75 | 68 | 66 | 53 | 80 | 125 | 43 | 39 | 33 | 33 | 55 | 211 | 70 | 38 |

As can be seen from the results presented in Table 2, the dental compositions of Examples had an ease of polishing of 55% or more, a shear adhesive strength of 7 MPa or more to enamel, a shear adhesive strength of 6 MPa or more to dentin, a flexural strength of 100 MPa or more, and a shear viscosity of 33 to 125 s⁻¹, demonstrating high mechanical strength, excellent ease of polishing, and favorable paste properties, as well as high adhesive strength to tooth structure.

In Comparative Examples, aggregate filler (d-1) is absent in Comparative Examples 1 to 3.

Comparative Example 1 containing the filler 6 with a large average primary particle diameter of 2.2 µm exhibited considerably low ease of polishing at 4%.

Comparative Example 2 with filler 7 exhibited ease of polishing in the preferred range. However, its high shear viscosity value of 211 s⁻¹ led to low monomer flowability, and the adhesive strength to dentin was low at 3 MPa.

Comparative Example 3 with filler 8 exhibited favorable ease of polishing. However, the shear adhesive strength to dentin and the flexural strength were low, 4 MPa and 82 MPa, respectively.

In Comparative Example 4 in which the monomer (a) having an acidic group was absent, the shear adhesive strength was merely 1 MPa for both enamel and dentin.

### INDUSTRIAL APPLICABILITY

A dental composition according to the present invention can be suitably used in the field of dental treatment as self-adhesive dental composite resins or dental cements.

## Claims

1. A dental composition comprising a monomer (a) having an acidic group, a monomer (b) having no acidic group, a polymerization initiator (c), and a filler (d),
wherein the filler (d) comprises an aggregate filler (d-1) in which primary particles are aggregated,
the primary particles are metal oxides, and
the aggregate filler (d-1) has a specific surface area of 5 m²/g or more and 60 m²/g or less as measured by a BET method.

2. The dental composition according to claim 1, wherein the primary particles of the aggregate filler (d-1) have an average particle diameter of 30 to 500 nm.

3. The dental composition according to claim 1 or 2, which has a viscosity of 1 to less than 200 Pa·s as measured with a rotational viscoelasticity meter at 25°C with a shear rate of 10 s⁻¹.

4. The dental composition according to claim 1 or 2, wherein the filler (d) further comprises a filler (d-2) other than the aggregate filler (d-1).

5. The dental composition according to claim 1 or 2, wherein the metal oxides forming the primary particles of the aggregate filler (d-1) comprise Si and Zr elements.

6. The dental composition according to claim 1 or 2, wherein the polymerization initiator (c) comprises a water-soluble photopolymerization initiator (c-1a).

7. The dental composition according to claim 1 or 2, wherein the content of the aggregate filler (d-1) is 20 to 85 mass% in total 100 mass% of the dental composition.

8. The dental composition according to claim 1 or 2, wherein the content of the monomer (a) having an acidic group is 0.1 to 25 mass% in total 100 mass% of the dental composition.

9. The dental composition according to claim 1 or 2, wherein the content of the filler (d) is 50 to 90 mass% in total 100 mass% of the dental composition.

10. The dental composition according to claim 1 or 2, wherein the aggregate filler (d-1) has an average particle diameter of 1 to 20 µm in terms of secondary particles.

11. The dental composition according to claim 1 or 2, wherein the aggregate filler (d-1) has a specific surface area of 5 m²/g or more and less than 50 m²/g as measured by a BET method.

12. A self-adhesive dental composite resin comprising a dental composition of claim 1 or 2.

13. A dental cement comprising a dental composition of claim 1 or 2.

14. A method for producing a dental composition of claim 1 or 2, comprising mixing at least three of the monomer (a) having an acidic group, the monomer (b) having no acidic group, the polymerization initiator (c), and the aggregate filler (d-1).

15. The method for producing a dental composition according to claim 14, which further comprises mixing a filler (d-2) other than the aggregate filler (d-1), and a polymerization accelerator (e).
